# EUROPÄISCHE PATENTSCHRIFT

(11) **EP 3 025 667 B1**
(45) Veröffentlichungstag und Bekanntmachung des Hinweises auf die Patenterteilung: **14.06.2017**
(21) Anmeldenummer: 15196110.9
(22) Anmeldetag: 24.11.2015
(51) Int. Cl.: A61B 34/30, A61B 46/10, A61B 34/37

(54) **VORRICHTUNG ZUR ROBOTERGESTÜTZTEN CHIRURGIE**
DEVICE FOR ROBOTIC SURGERY
DISPOSITIF DE CHIRURGIE ROBOTISE

(30) Priorität: 27.11.2014 DE 102014117408
(43) Veröffentlichungstag der Anmeldung: 01.06.2016
(73) Patentinhaber: avateramedical GmbH, 07745 Jena (DE)
(72) Erfinder: Karguth, Andreas, 99869 Tüttleben (DE); Trommer, Christian, 99310 Wipfratal/Schmerfeld (DE); Seeber, Marcel, 07745 Jena (DE)
(74) Vertreter: Schaumburg und Partner Patentanwälte mbB

(56) Entgegenhaltungen:
- WO-A1-2013/159932
- WO-A1-2014/162217
- WO-A2-2013/067421
- WO-A2-2014/005689
- US-A1- 2006 161 138

## Beschreibung

Die Erfindung betrifft eine Vorrichtung zur robotergestützten Chirurgie mit mindestens in einem nicht sterilen Bereich angeordneten Manipulatorarm mit einer Koppeleinheit, die mindestens ein erstes Übertragungsmittel hat. Die Vorrichtung hat mindestens eine in einem sterilen Bereich angeordnete Sterileinheit, die mindestens ein zweites Übertragungsmittel hat und eine sterile Abdeckung zum Abschirmen des Manipulatorarms von dem sterilen Bereich.

In der minimal invasiven Chirurgie werden zunehmend sogenannte Telemanipulatorsysteme, die auch als Roboterassistenzsysteme bezeichnet werden, eingesetzt. Das sterile Operationsfeld wird vor den nicht sterilen Elementen des Telemanipulatorsystems mit Hilfe einer sterilen Abdeckung geschützt. Durch die sterile Abdeckung wird sowohl eine Kontamination des sterilen Operationsfeldes als auch einer Verschmutzung des Telemanipulatorsystems durch Körperflüssigkeiten und/oder Gewebe des operierten Patienten oder des Operationspersonals verhindert. Dadurch wird das Risiko von Kreuzkontaminationen verringert.

Mit Hilfe des Telemanipulatorsystems werden chirurgische Instrumente und/oder Endoskope aufgrund von Bedieneingaben in ihrer Lage und Orientierung gesteuert und kommen dabei zwangsläufig in Körperkontakt mit dem zu operierenden Patienten, sodass die chirurgischen Instrumente und/oder Endoskope mit Körperflüssigkeiten und/oder Gewebe des operierten Patienten verunreinigt werden. Gleichzeitig müssen die chirurgischen Instrumente mechanisch, elektrisch und/oder optisch an das Telemanipulatorsystem angekoppelt werden, um eine aktive Positionierung und Ausrichtung des chirurgischen Instruments sowie eine gewünschte Betätigung eines chirurgischen Instruments realisieren zu können. Hierzu haben die chirurgischen Instrumente, Endoskope oder zu bedienende medizinische Geräte eine Koppelschnittstelle, die als Koppeleinheit ausgebildet sein kann und auch als Sterileinheit bezeichnet wird.

Das während eines chirurgischen Eingriffs genutzte Material einschließlich der verwendeten chirurgischen Apparate und Instrumente und die weiteren Bestandteile des Telemanipulatorsystems können in drei Kategorien eingeteilt werden:
Kategorie 1: Das Material ist steril und wird während des chirurgischen Eingriffs kontaminiert. Das Material wird nach der Operation entsorgt. Es erfolgt somit eine Einmalverwendung des Materials.
Kategorie 2: Das Material ist steril, wird während des chirurgischen Eingriffs kontaminiert und nach der Operation gereinigt und sterilisiert. Es erfolgt somit eine Mehrfachverwendung des Materials. Solche mehrfach verwendeten Materialien müssen entsprechend den Anforderungen an eine prozessfähige Sterilisierbarkeit konstruiert und produziert sein.
Kategorie 3: Das Material ist nicht steril. Während des chirurgischen Eingriffs wird durch eine sterile Abdeckung und Umverpackung eine Kontamination des sterilen Operationsfeldes verhindert. Gleichzeitig wird das nicht sterile Material vor Kontakt mit Körperflüssigkeiten und/oder Gewebe geschützt.

Ist es erforderlich, Geräte der Kategorie 1 oder der Kategorie 2 mit Geräten der Kategorie 3 zu koppeln, ist eine Sterilschnittstelle erforderlich, die eine Kontamination der Geräte der Kategorie 1 bzw. der Kategorie 2 durch die nicht sterilen Geräte der Kategorie 3 verhindert und umgekehrt eine Verunreinigung der Geräte der Kategorie 3 verhindert, da diese im allgemeinen als nicht sterilisierbare autoklavierbare Komponenten technisch ausgeführt sind. Die Ausführung von Geräten als sterilisierbare und autoklavierbare Komponenten erfordert eine besondere technische Auslegung des Geräts für den Sterilisationsprozess, sodass hierfür ein höherer Entwicklungsaufwand sowie ein erheblicher Validierungsaufwand zum Nachweis der Wirksamkeit des Sterilisationsprozesses erforderlich sind. Für einen solchen Nachweis ist es insbesondere erforderlich, das Gerät mehrfach nacheinander zu kontaminieren, zu sterilisieren, eine Wirksamkeitsprüfung der Sterilisation durchzuführen sowie eine Funktionsprüfung nach erfolgter Sterilisation durchzuführen. Dabei ist ein Nachweis zu erbringen, dass die Geräte nach jeder Sterilisation sicher sterilisiert und somit wieder verwendet werden könnten.

Aus dem Dokument US 7,666,191 B1 ist ein Telemanipulatorsystem bekannt, bei dem die nicht sterilen Manipulatorarme mittels einer Sterilfolie abgedeckt werden. Die Koppeleinheit des Manipulatorarms umfasst vier Rotationsaktuatoren, die mit einer ersten Seite eines in der Sterilfolie integrierten Steriladapters gekoppelt werden. Mit Hilfe des Steriladapters werden die Drehbewegungen der vier Rotationsaktuatoren der Koppeleinheit des Manipulatorarms in Eingriff mit vier in den Steriladapter integrierten drehbar gelagerten Übertragungsmittel gekoppelt. An der sterilen Außenseite des Steriladapters können diese an der Außenseite des Steriladapters sterilen Übertragungsmittel mit angetriebenen Elementen des sterilen chirurgischen Instruments in Eingriff gebracht werden. Ferner können über diesen Steriladapter elektrische Signale zwischen der Innenseite und der Außenseite des Steriladapters übertragen werden.

Somit wird mit Hilfe des Steriladapters verhindert, dass die Rotationsaktuatoren und die elektrischen Anschlüsse des sterilen chirurgischen Instruments direkt in Kontakt mit den Rotationsaktuatoren und den elektrischen Anschlüssen der Koppeleinheit des nicht sterilen Manipulatorarms kommen. Eine Kontamination des chirurgischen Instruments durch den Kontakt mit nicht sterilen Teilen des Manipulatorarms wird durch den Steriladapter verhindert. Bei dieser Lösung ist es jedoch erforderlich, dass der Steriladapter drehbar gelagerte Übertragungsmittel haben muss sowie Übertragungsmittel zur Übertragung von elektrischen Signalen, wodurch der Adapter aufwendig herzustellen und störanfällig ist. Insbesondere ist es aufwendig, die Drehbarkeit der Übertragungsmittel sicherzustellen, wenn die Übertragungsmittel in Kontakt mit Körperflüssigkeit kommen.

Grundsätzlich ist jedes Element in der Funktionskette zur Kopplung des Manipulatorarms und des Instruments eine mögliche Fehlerquelle und ist mit zusätzlichen Kosten verbunden. Der Steriladapter selbst ist als Teil der Sterilfolie zur Einmalverwendung vorgesehen.

Aus dem Dokument US 8,074,657 B2 ist ein weiterer Steriladapter bekannt, der eine Aktuatoreinheit zur Übertragung mechanischer Energie auf ein mit dem Steriladapter gekoppelten chirurgischen Instrument ermöglicht.

Aufgabe der vorliegenden Erfindung ist es, eine Vorrichtung und eine Anordnung zur robotergestützten Chirurgie anzugeben, bei denen eine sterile Kopplung eines in einem nicht sterilen Bereich angeordneten Manipulatorarms mit einer in einem sterilen Bereich angeordneten Sterileinheit einfach möglich ist, wobei durch die Kopplung eine zuverlässige mechanische Kraft- und Momentenübertragung und/oder optische Übertragung und/ oder elektrische Signal und/oder Energieübertragung zwischen dem Manipulatorarm und der Instrumenteneinheit, die insbesondere eine Sterileinheit und ein mit dieser verbundenes chirurgisches Instrument umfasst, ermöglicht wird.

Diese Aufgabe wird durch eine Vorrichtung zur robotergestützten Chirurgie mit den Merkmalen des Anspruchs 1 und durch eine Anordnung mit den Merkmalen des Anspruchs 15 gelöst. Vorteilhafte Weiterbildungen der Erfindung sind in den abhängigen Ansprüchen angegeben.

Durch das Vorsehen eines translatorischen Antriebselements zum Erzeugen einer translatorischen Antriebsbewegung und eines rotatorischen Antriebselements zum Erzeugen einer rotatorischen Antriebsbewegung in der Koppeleinheit und durch das Vorsehen eines mit dem translatorischen Antriebselement koppelbaren translatorischen angetriebenen Elements und eines mit dem rotatorischen Antriebselement koppelbaren rotatorischen angetriebenen Elements ist ein einfacher Antrieb der Instrumenteneinheit, die insbesondere eine Sterileinheit und ein mit dieser verbundenes chirurgisches Instrument umfasst, möglich. Durch die in der sterilen Abdeckung vorhandene Sterilschleuse ist wiederum eine sichere von der Koppeleinheit unabhängige sterile Abschirmung der Antriebselemente vorzugsweise möglich, wenn die Sterileinheit nicht mit der Sterilschleuse verbunden ist. Die Sterilschleuse schirmt die Antriebselemente auch ab, nachdem die Sterileinheit von der Sterilschleuse getrennt worden ist. Dadurch ist eine sichere sterile Abdeckung der nicht sterilen Elemente des Manipulatorarms gegenüber dem sterilen Bereich einfach möglich.

Mithilfe der rotatorischen Antriebseinheit und der translatorischen Antriebseinheit sind beispielsweise das Drehen eines Instruments und das Abwinkeln der Instrumentenspitze oder das Drehen des Instruments und eine Fassbewegung einfach ausführbar. Alternativ kann der Instrumentenschaft gedreht und eine Fassbewegung des Instruments ausgeführt werden.

Besonders vorteilhaft ist es, wenn mithilfe des mit dem translatorischen angetriebenen Element gekoppelten translatorischen Antriebselements die translatorische Antriebsbewegung zum translatorischen angetriebenen Element übertragbar ist. Mithilfe des mit dem rotatorischen angetriebenen Element gekoppelten rotatorischen Antriebselements ist die rotatorische Antriebsbewegung zum rotatorischen angetriebenen Element übertragbar.

Ein rotatorisches Antriebselement ist allgemein ein Antriebselement, das eine rotatorische Antriebsbewegung bzw. eine Rotationsbewegung ausführen kann und dazu insbesondere von einem Antriebsmotor direkt oder indirekt angetrieben wird.

Ein rotatorisch angetriebenes Element ist allgemein ein mit dem rotatorischen Antriebselement koppelbares angetriebenes Element, das eine rotatorische Bewegung bzw. eine Rotationsbewegung ausführt, wenn das mit dem angetriebenen rotatorischen Element gekoppelte rotatorische Antriebselement eine rotatorische Antriebsbewegung bzw. Rotationsbewegung ausführt.

Ein translatorisches Antriebselement ist allgemein ein Antriebselement, das eine translatorische Antriebsbewegung bzw. eine Translationsbewegung ausführen kann und dazu insbesondere von einem Antriebsmotor direkt oder indirekt angetrieben wird.

Ein translatorisch angetriebenes Element ist allgemein ein mit einem translatorischen Antriebselement koppelbares angetriebenes Element, das eine translatorische Bewegung bzw. Translationsbewegung ausführt, wenn das mit dem angetriebenen translatorischen Element gekoppelte translatorische Antriebselement eine translatorische Antriebsbewegung bzw. Translationsbewegung ausführt. Beim Koppeln eines Antriebselements mit einem angetriebenen Element erfolgt vorzugsweise ein direkter Kontakt jeweils eines Antriebselements mit einem komplementären angetriebenen Element zur Übertragung der Antriebsbewegung. Dies kann insbesondere durch eine formschlüssige und/oder kraftschlüssige Verbindung und/oder magnetische Kopplung zwischen dem jeweiligen angetriebenen Element und dem Antriebselement erfolgen.

Vorteilhaft ist es, wenn die mit der Koppeleinheit und mit der Sterileinheit verbundene Sterilschleuse einen Zugang zu den Antriebselementen derart freigibt, dass das translatorische Antriebselement mit dem translatorischen angetriebenen Element und das rotatorische Antriebselement mit dem rotatorischen angetriebenen Element koppelbar ist. Eine Kopplung zwischen einem Antriebselement und einem angetriebenen Element erfolgt insbesondere durch direkten Eingriff, wie z.B. eine formschlüssige Verbindung, oder eine kraftschlüssige Verbindung.

Ferner ist es vorteilhaft, wenn die Sterileinheit eine sterile Abdeckung umfasst, die die angetriebenen Elemente steril abschirmt, bevor die Sterileinheit mit der Sterilschleuse verbunden ist und vorzugsweise nachdem die Sterileinheit von der Sterilschleuse getrennt worden ist. Die Sterilschleuse gibt den Zugang zu den angetriebenen Elementen frei, wenn die Sterilschleuse mit der Koppeleinheit und mit der Sterileinheit verbunden ist. Dadurch ist eine einfache Handhabung des chirurgischen Elements und des Manipulatorarms möglich, da die Sterileinheit mit dem chirurgischen Instrument einfach von der Koppeleinheit des Manipulatorarms verbindbar und wieder trennbar ist, wobei sichergestellt ist, dass die angetriebenen Elemente steril abgeschirmt sind, wenn die Sterileinheit nicht mit der Sterilschleuse verbunden bzw. nicht mit der Koppeleinheit gekoppelt ist. Dadurch ist eine sichere Handhabung der Instrumenteneinheit im sterilen Bereich möglich, so dass die Instrumenteneinheit auch nach einem Kontakt der angetriebenen Elemente mit den nicht sterilen Antriebselementen im sterilen Bereich abgelegt werden kann, ohne dass der sterile Bereich dadurch kontaminiert wird.

Ferner ist es vorteilhaft, wenn die Sterilschleuse eine sterile Abdeckung umfasst, die die Antriebselemente steril abschirmt, bevor die Sterileinheit mit der Sterilschleuse verbunden ist und vorzugsweise nachdem die Sterileinheit von der Sterilschleuse getrennt worden ist. Die Sterilschleuse gibt den Zugang zu den Antriebselementen frei, wenn die Sterilschleuse mit der Koppeleinheit und mit der Sterileinheit verbunden ist. Dadurch ist sichergestellt, dass die üblicherweise nicht sterilen Antriebselemente der Koppeleinheit mithilfe der Sterilschleuse immer dann sicher vom sterilen Bereich abgeschirmt sind, wenn die Sterileinheit der Instrumenteneinheit nicht mit der Sterilschleuse verbunden ist, insbesondere nachdem die Sterileinheit der Instrumenteneinheit wieder von der Instrumentenschleuse getrennt worden ist. Die Sterilschleuse gibt den Zugang zu den Antriebselementen frei, wenn die Sterilschleuse mit der Koppeleinheit und mit der Sterileinheit verbunden ist. Hierdurch wird eine sichere Abschirmung der nicht sterilen Koppeleinheit, insbesondere der nicht sterilen Antriebselemente, gegenüber dem sterilen Bereich erreicht. Beim Koppeln der Sterileinheit mit der Sterilschleuse wird vorzugsweise eine steril abgedeckte Zugangsöffnung der Sterilschleuse freigegeben, damit die Antriebselemente mit den angetriebenen Elementen miteinander in direkten Kontakt gebracht werden können, insbesondere in direkten Eingriff miteinander gebracht werden können.

Die sterile Abdeckung der Sterilschleuse zum sterilen Abdecken der Antriebselemente und/oder die sterile Abdeckung der Sterileinheit zum sterilen Abschirmen der angetriebenen Elemente kann eine Jalousie, ein Rollo, eine mit einer Öffnung versehene drehbare Scheibe und/oder eine Klappe umfassen. Dadurch ist ein einfaches sicheres Verschließen und Öffnen mindestens einer Zugangsöffnung zu den Antriebselementen bzw. den angetriebenen Elementen einfach möglich.

Besonders vorteilhaft ist es, wenn das translatorische Antriebselement ein erstes translatorisches Antriebselement und das rotatorische Antriebselement ein erstes rotatorisches Antriebselement ist und wenn das translatorische angetriebne Element ein erstes translatorisches angetriebenes Element und wenn das rotatorische angetriebene Element ein erstes rotatorisches angetriebenes Element ist. Die Koppeleinheit umfasst ein zweites translatorisches Antriebselement zum Erzeugen einer translatorischen Antriebsbewegung und ein zweites rotatorisches Antriebselement zum Erzeugen einer rotatorischen Antriebsbewegung. Die Sterileinheit hat ein mit dem zweiten translatorischen Antriebselement koppelbares zweites translatorisches angetriebenes Element und ein mit dem zweiten rotatorischen Antriebselement koppelbares zweites rotatorisches angetriebenes Element. Die mit der Koppeleinheit verbundene Sterilschleuse schirmt das erste und das zweite translatorische Antriebselement und das erste und das zweite rotatorische Antriebselement steril ab, bevor die Sterileinheit mit der Sterilschleuse verbunden ist und vorzugsweise nachdem die Sterileinheit von der Sterilschleuse getrennt worden ist.

Allgemein sind die Antriebsbewegungen aller Antriebselemente unabhängig voneinander, insbesondere nicht mechanisch gekoppelt. Alle Antriebsbewegungen der Antriebselemente werden mit Hilfe einer Steuereinheit gesteuert und vorzugsweise elektronisch überwacht.

Weiterhin ist es vorteilhaft, wenn das erste und/oder zweite translatorische angetriebene Element mithilfe der Rückstellkraft eines elastisch verformbaren Elements in eine Ausgangsposition bewegbar ist und wenn diese Rückstellkraft das jeweilige translatorische angetriebene Element nach dem Trennen der Sterileinheit von der Sterilschleuse in seine Ausgangsposition bewegt. Dadurch ist eine definierte Lage der translatorischen angetriebenen Elemente beim Verbinden bzw. wieder Verbinden der Sterileinheit mit der Sterilschleuse und bei der dadurch erfolgten Kopplung der Antriebselemente mit den angetriebenen Elementen, einfach möglich. Dabei ist die Rückstellkraft zumindest so groß, dass sie die durch die translatorischen Antriebselemente bewirkte Verstellung des chirurgischen Instruments in einer Ausgangslage zurückstellt, wenn keine weiteren Krafteinwirkungen auf das Instrument die Rückstellung behindern oder verhindern.

Weiterhin ist es vorteilhaft, wenn die Koppeleinheit mindestens einen Positionssensor zum Detektieren mindestens einer Drehwinkelposition eines ersten und/oder eines zweiten rotatorisch angetriebenen Elements hat. Vorzugsweise umfasst die Vorrichtung einen ersten Positionssensor zum Detektieren mindestens einer Drehwinkelposition des ersten rotatorisch angetriebenen Elements und einen zweiten Positionssensor zum Detektieren mindestens einer Drehwinkelposition des zweiten rotatorisch angetriebenen Elements. Bei einer Initialisierung wird dann das erste rotatorisch angetriebene Element mithilfe des ersten rotatorischen Antriebselements so lang gedreht, bis der erste Positionssensor eine definierte Winkelstellung des angetriebenen rotatorischen Elements detektiert. Insbesondere umfasst das angetriebene rotatorische Element an einer Winkelposition einen mithilfe des ersten Positionssensors detektierbaren Vorsprung oder eine Vertiefung, der bzw. die mithilfe des Positionssensors detektierbar ist, wenn der Vorsprung bzw. die Vertiefung dem Positionssensor gegenüberliegend angeordnet sind. Damit kann die Winkellage des rotatorisch angetriebenen Elements detektiert und davon ausgehend der weitere Antrieb des angetriebenen rotatorischen Elements mithilfe einer Steuereinheit derart überwacht werden, dass die Winkelposition des angetriebenen rotatorischen Elements zu jedem Zeitpunkt bekannt ist und bei der weiteren Ansteuerung berücksichtigt wird.

In gleicher Weise kann eine definierte Drehwinkelposition des zweiten rotatorisch angetriebenen Elements mithilfe des zweiten Positionssensors detektiert werden, so dass mithilfe einer Steuereinheit der Antrieb des zweiten rotatorisch angetriebenen Elements derart überwacht wird, dass zu jedem Zeitpunkt dessen Drehwinkelposition bekannt ist und bei der weiteren Ansteuerung berücksichtigt werden kann.

Weiterhin ist es vorteilhaft, wenn die Koppeleinheit das translatorische Antriebselement in einer Ausgangsposition bewegt, nachdem die Sterileinheit von der Sterilschleuse getrennt worden ist. Dadurch kann das in seiner Ausgangsposition angeordnete translatorische Antriebselement mit dem in seiner Ausgangsposition angeordneten translatorischen angetriebenen Element beim Verbinden der Sterileinheit mit der mit der Koppeleinheit verbundenen Sterilschleuse automatisch in direkten Eingriff gebracht werden.

Ein translatorisches Antriebselement kann beispielsweise durch eine Gabel gebildet sein, deren Zinken in einer umlaufenden Nut des translatorischen angetriebenen Elements eingreift bzw. eingreifen. Diese Nut ist vorzugsweise eine umlaufende Nut, so dass das translatorische angetriebene Element zusammen mit Elementen der Instrumenteneinheit zur rotatorischen Kraftübertragung, wie z. B. einem Instrumentenschafft, in der Gabel gedreht werden können.

Besonders vorteilhaft ist es, wenn die Koppeleinheit das erste translatorische Antriebselement in seine Ausgangsposition bewegt, nachdem die Sterileinheit von der Sterilschleuse getrennt worden ist. Das in seiner Ausgangsposition angeordnete erste translatorische Antriebselement ist mit dem in seiner Ausgangsposition angeordneten ersten translatorischen angetriebenen Element beim Verbinden der Sterileinheit mit der mit der Koppeleinheit verbundenen Sterilschleuse automatisch in direkten Eingriff bringbar.

Ferner ist es vorteilhaft, wenn die Koppeleinheit das zweite translatorische Antriebselement in seine Ausgangsposition bewegt, nachdem die Sterileinheit von der Sterilschleuse getrennt worden ist. Das in seiner Ausgangsposition angeordnete zweite translatorische Antriebselement ist mit dem in seiner Ausgangsposition angeordneten zweiten translatorischen angetriebenen Element beim Verbinden der Sterileinheit mit der mit der Koppeleinheit verbundenen Sterilschleuse automatisch in direkten Eingriff bringbar.

Somit ist es besonders vorteilhaft, wenn das erste und/oder zweite translatorisch angetriebene Element mithilfe des elastisch verformbaren Elements nach dem Trennen der Sterileinheit von der Sterilschleuse in seine Ausgangsposition bewegt wird und wenn das erste und/oder zweite translatorische Antriebselement nach dem Trennen der Sterileinheit von der Sterilschleuse mithilfe der Antriebselement in seine Ausgangsposition bewegt wird. Hierdurch ist sichergestellt, dass ein erneutes Verbinden der Sterileinheit mit der Sterilschleuse und die dadurch bewirkte Kopplung der Antriebselemente mit den angetriebenen Elementen problemlos möglich ist.

Weiterhin ist es vorteilhaft, wenn die angetriebenen Elemente entlang der Längsachse des chirurgischen Instruments nacheinander angeordnet sind, und wenn die angetriebenen Elemente von dem proximalen Ende des chirurgischen Instruments vorzugsweise in der Reihenfolge erstes rotatorisches angetriebenes Element, erstes translatorisches angetriebenes Element, zweites rotatorisches angetriebenes Element, zweites translatorisches angetriebenes Element angeordnet sind. Optional können in der Reihenfolge nachfolgend elektrische Kontakte und/oder eine optische Schnittstelle entlang der Längsachse angeordnet sein. Im verbundenen Zustand der Sterileinheit mit der Sterilschleuse und der Sterilschleuse mit der Koppeleinheit sind den angetriebenen Elementen komplementäre Antriebselemente, den elektrischen Kontakten komplementäre elektrische Kontakte und der optischen Schnittstelle eine komplementäre optische Schnittstelle gegenüberliegend angeordnet. Dadurch ist ein kompakter und kostengünstiger Aufbau sowohl der Sterileinheit als auch der Koppeleinheit möglich.

Ferner ist es vorteilhaft, wenn das erste rotatorische angetriebene Element drehfest mit dem äußeren Instrumentenschaft zu dessen Drehung verbunden ist. Dadurch ist eine Drehung des äußeren Instrumentenschafts um dessen Längsachse durch das erste rotatorische Antriebselement über das erste rotatorische angetriebene Element einfach möglich.

Ferner kann das erste translatorische angetriebene Element mit einem in dem äußeren Instrumentenschaft in Richtung der Längsachse des äußeren Instrumentenschafts längs verschiebbar angeordneten ersten inneren Instrumentenschaft verbunden sein, wobei bei einer Bewegung des ersten inneren Instrumentenschafts die Instrumentenspitze um eine Drehachse verschwenkbar ist, die vorzugsweise orthogonal zur Längsachse des chirurgischen Instruments verläuft. Dadurch kann insbesondere eine am proximalen Ende des chirurgischen Instruments angeordnete Instrumentenspitze bei einem Antrieb des translatorischen angetriebenen Elements mithilfe der ersten translatorischen Antriebseinheit abgewinkelt werden.

Ferner kann das zweite rotatorische angetriebene Element drehfest mit einem in dem ersten inneren Instrumentenschaft angeordneten zweiten inneren Instrumentenschaft drehfest verbunden sein, wobei die Drehung des zweiten inneren Instrumentenschafts eine Rotation des Endeffektors unabhängig von dem äußeren Instrumentenschaft erfolgt. Anstatt des zweiten inneren Instrumentenschafts oder in dem zweiten inneren Instrumentenschaft kann eine Dreh- und Schubstange vorgesehen sein, die insbesondere elastisch verformbar und beispielsweise als Drahtseil ausgebildet sein kann.

Das zweite translatorische angetriebene Element kann mit dem relativ zum ersten inneren Instrumentenschaft längs verschiebbar angeordneten zweiten inneren Instrumentenschaft zum Betätigen des Endeffektors verbunden sein. Dadurch kann insbesondere eine Betätigung des Endeffektors der Instrumentenspitze einfach erfolgen.

Die Drehachse, um die der Endeffektor verschwenkbar ist, ist vorzugsweise orthogonal zur Längsachse der inneren und des äußeren Instrumentenschäfte. Vorzugsweise sind die inneren und der äußere Instrumentenschaft drehfest zueinander. Ferner haben der äußere Instrumentenschaft und die inneren Instrumentenschäfte dieselbe Längsachse, d.h. sie sind koaxial.

Ferner ist es vorteilhaft, wenn die Sterileinheit mindestens einen ersten koaxial zum zweiten inneren Instrumentenschaft mit diesem elektrisch leitend verbundenen Schleifring hat. Hierdurch kann elektrische Energie von der Koppeleinheit über den Schleifring zum Endeffektor geleitet werden, um beispielsweise ein monopolares chirurgisches Instrument zur Hochfrequenzchirurgie mit elektrischer Energie zu versorgen.

Besonders vorteilhaft ist es, wenn die Sterileinheit einen zweiten koaxial zum ersten inneren Instrumentenschaft mit diesem elektrisch verbundenen Schleifring hat, wobei der zweite innere Instrumentenschaft und der erste innere Instrumentenschaft elektrisch isoliert zueinander angeordnet sind. Vorzugsweise sind auch der erste innere Instrumentenschaft und der äußere Instrumentenschaft elektrisch isoliert zueinander angeordnet. Hierdurch kann auch eine Übertragung von elektrischer Energie für ein bipolares chirurgisches Instrument sicher mit elektrischer Energie versorgt werden.

Besonders vorteilhaft ist es, wenn das chirurgische Instrument einen Endeffektor mit zwei mithilfe des zweiten inneren Instrumentenschafts relativ zueinander bewegbaren Arme hat und wenn die Arme elektrisch isoliert zueinander angeordnet sind. Die Arme sind jeweils mit einem elektrischen Kabel elektrisch leitend verbunden, die im zweiten inneren Instrumentenschaft oder in einem dritten in dem zweiten inneren Instrumentenschaft angeordneten Instrumentenschaft geführt sind. Das Kabel ist vorzugsweise ein isolierter Draht zur Übertragung von elektrischer Energie zwischen der Sterileinheit dem Endeffektor. Hierdurch ist eine einfache und sichere Übertragung von hochfrequenter elektrischer Energie von der Sterileinheit zur Instrumentenspitze bzw. zum Endeffektor einfach möglich. Ferner kann ein erster Arm des Endeffektors kann zum Erzeugen der relativen Bewegung der Arme zueinander aktiv bewegt werden und ein zweiter Arm des Endeffektors feststehen. Die Arme sind vorzugsweise Greifarme, Klemmarme und können jeweils eine Schneide und/oder einen Klemmbereich umfassen.

Ferner ist es vorteilhaft, wenn die Sterileinheit ein erstes Lager zum drehbaren Lagern des äußeren Instrumentenschafts gegenüber einem mit der Sterilschleuse koppelbaren Gehäuse der Sterileinheit hat und wenn die Sterileinheit mindestens ein zweites Lager zum drehbaren Lagern der Dreh- und Schubstange gegenüber dem Gehäuse der Sterileinheit hat. Hierdurch ist eine einfache und sichere Lagerung der Elemente des Instrumentenschafts und der angetriebenen Elemente einfach möglich.

Ferner ist es vorteilhaft, wenn die Koppeleinheit über Stromversorgungsleitungen mit einer Ansteuerschaltung einer Steuereinheit und/oder einer Stromversorgungseinheit, vorzugsweise über mindestens einen Steckverbinder, zur Versorgung mindestens einer in der Koppeleinheit angeordneten elektrischen Antriebseinheit verbunden ist. Vorzugsweise sind in der Koppeleinheit vier elektrische Antriebseinheiten angeordnet, die jeweils ein Antriebselement, wie das erste rotatorische Antriebselement, das zweite rotatorische Antriebselement, das erste translatorische Antriebselement und/oder das zweite translatorische Antriebselement antreiben.

Ferner kann die Steuereinheit über mindestens eine Steuer- und/oder Signaleinheit, vorzugsweise über einen Steckverbinden, zur Kopplung der Steuereinheit mit der Koppeleinheit verbunden sein. Auch kann die Koppeleinheit mit mindestens eine Stromversorgungsleitung zum Zuführen hochfrequenter elektrischer Energie für die Hochfrequenzchirurgie verbunden sein. Die hochfrequente elektrische Energie wird vorzugsweise über Schleifringkontakte der Koppeleinheit zu den Schleifringen der Sterileinheit übertragen. Hierdurch ist eine einfache Verwendung von chirurgischen Instrumenten zur Hochfrequenzchirurgie möglich, wobei die Stromversorgung des chirurgischen Instruments zur Hochfrequenzchirurgie ohne separate Verbindungen einfach durch Koppeln der Sterileinheit mit der Koppeleinheit erfolgt.

Weiterhin können die Sterileinheit und die Koppeleinheit Übertragungsmittel zur optischen und/oder elektrischen Übertragung von Mess- und/oder Steuersignalen und/oder Bildinformationen, zur Bereitstellung eines optischen Kanals zur Beleuchtung und/oder zur Bildübertragung zwischen der Sterileinheit und der Koppeleinheit haben.

Bei der Erfindung wird insbesondere bei einer Verbindung der Sterileinheit mit der Sterilschleuse die zuvor steril abgedeckten Antriebselemente für eine Koppelung mit den angetriebenen Elementen freigegeben. Beim Trennen der Sterileinheit von der Sterilschleuse wird zumindest das erste Übertragungsmittel und wieder steril abgeschirmt. Vorzugsweise ist die Sterilschleuse beim Verbinden und Trennen der Sterileinheit mit bzw. von der Sterilschleuse bereits mit der Koppeleinheit verbunden. Vorzugsweise bleibt die Sterilschleuse über den gesamtem Zeitraum des chirurgischen Eingriffs mit der Koppeleinheit verbunden, wobei die Instrumenteneinheit mit der Sterileinheit mehrfach von der Koppeleinheit des Manipulatorarms getrennt und wieder mit diesen verbunden bzw. gegen eine weitere Instrumenteneinheit mit einer weiteren Sterileinheit ausgetauscht werden kann.

Durch die Erfindung ist es insbesondere möglich, die Sterilschleuse ohne mechanische und/oder elektrische Übertragungsmittel auszustatten, sodass sowohl eine sichere sterile Abschirmung des nicht sterilen Manipulatorarms und der nicht sterilen Koppeleinheit als auch eine sichere Kopplung der Antriebseinheiten mit den angetriebenen Einheiten ohne Zwischenschaltung weiterer Übertragungsmittel, insbesondere ohne Zwischenschaltung weiterer mechanischer Übertragungsmittel, möglich ist. Die sterile Abdeckung umfasst insbesondere ein steriles flexibles Material, wie eine Sterilfolie, und die mindestens eine Sterilschleuse.

Es ist vorteilhaft, wenn die Sterileinheit mindestens eine Sterilklappe hat, die in einem geschlossenen Zustand das zweite Übertragungsmittel steril abschirmt. Beim Verbinden der Sterileinheit mit der Sterilschleuse erfolgt dann jeweils eine Bewegung der Schleusenklappe und der Sterilklappe vom geschlossenen Zustand in den geöffneten Zustand, sodass eine direkte Übertragung zwischen dem ersten Übertragungsmittel und dem zweiten Übertragungsmittel durch eine von der Schleusenklappe und der Sterilklappe im geöffneten Zustand freigegebene Öffnung möglich ist. Beim Trennen der Sterileinheit von der Sterilschleuse erfolgt eine Bewegung der Schleusenklappe und der Sterilklappe jeweils vom geöffneten Zustand in den geschlossenen Zustand, sodass die Schleusenklappe nach dem Trennen das erste Übertragungsmittel und die Sterilklappe nach dem Trennen das zweite Übertragungsmittel vom sterilen Bereich abschirmen.

Die Sterileinheit ist gemäß der Definition der Beschreibungseinleitung Material der Kategorie 1 und 2 und ist somit steril.

Ferner ist es vorteilhaft, wenn die Koppeleinheit mit einem ersten Verbindungsbereich der Sterilschleuse verbindbar ist und wenn die Sterileinheit mit einem zweiten Verbindungsbereich der Sterilschleuse verbindbar ist. Der erste Verbindungsbereich und der zweite Verbindungsbereich sind vorzugsweise auf voneinander abgewandten Seiten der Sterilschleuse angeordnet. Dadurch ist eine einfache Kopplung und somit eine einfache Handhabung sowohl der sterilen Abdeckung als auch der Sterileinheit vor, während und nach einem chirurgischen Eingriff möglich. Ferner ist es besonders vorteilhaft, wenn der zweite Verbindungsbereich als Aufnahmebereich ausgebildet ist, in dem die Sterileinheit beim Verbinden mit dem zweiten Verbindungsbereich zumindest teilweise aufnehmbar ist. Dadurch kann eine einfache und sichere Verbindung zwischen der Sterileinheit und der Sterilschleuse hergestellt werden. Insbesondere kann die Sterileinheit zumindest teilweise in den Aufnahmebereich hineingedrückt und dort verriegelt werden.

Ferner ist es vorteilhaft, wenn die Sterilschleuse einen dritten Verbindungsbereich hat, mit dem die flexible Abdeckung verbindbar ist, wobei der dritte Verbindungsbereich vorzugsweise umlaufend um die Sterilschleuse, insbesondere an der Mantelfläche umlaufend, vorzugsweise zwischen dem ersten und zweiten Verbindungsbereich, angeordnet ist. Durch die Sterilschleuse erfolgt eine einfache Verbindung des sterilen Bereichs und des nicht sterilen Bereichs zur Kopplung der Koppeleinheit mit der Sterileinheit, ohne dass die Sterileinheit derart kontaminiert wird, dass sie nach einer Trennung von der Sterilschleuse nicht mehr im sterilen Bereich verbleiben kann.

Vorzugsweise ist die Koppeleinheit am proximalen Ende des Manipulatorarms angeordnet.

Ferner ist es vorteilhaft, wenn der erste Verbindungsbereich der Sterilschleuse über eine erste lösbare Rastverbindung mit der Koppeleinheit verbindbar ist und der zweite Verbindungsbereich der Sterilschleuse über eine zweite lösbare Rastverbindung mit der Sterileinheit verbindbar ist. Dadurch ist die Sterilschleuse sowohl mit der Koppeleinheit als auch mit der Sterileinheit sicher verbindbar und einfach von diesen wieder trennbar, sodass eine einfache Handhabung sowohl der sterilen Abdeckung mit der Sterilschleuse als auch der Sterileinheit, insbesondere während einer Operation, möglich ist.

Besonders vorteilhaft ist es, wenn die Koppeleinheit mindestens einen Koppelsensor umfasst, der das Vorhandensein einer korrekt mit der Sterilschleuse verbundenen Sterileinheit detektiert. Ferner hat die Vorrichtung eine Steuereinheit, die eine Übertragung zwischen dem ersten Übertragungsmittel und dem zweiten Übertragungsmittel nur dann zulässt, wenn mit Hilfe des Koppelsensors eine korrekt mit der Sterilschleuse verbundene Sterileinheit detektiert worden ist. Bei einer weiteren vorteilhaften Ausführungsform detektiert der Koppelsensor mit Hilfe eines an der Sterileinheit vorgesehenen Detektionselements, das bei einer Verbindung mit der Sterilschleuse bis in den ersten Verbindungsbereich, mit dem die Koppeleinheit verbunden ist, ragt, dass sowohl die Sterileinheit mit dem zweiten Verbindungsbereich als auch die Koppeleinheit mit dem ersten Verbindungsbereich korrekt verbunden sind. Die Steuereinheit gibt einen Antrieb der angetriebenen Elemente durch die Antriebselemente vorzugsweise nur dann frei bzw. lässt diesen nur dann zu, wenn der Koppelsensor eine korrekte Verbindung zwischen der Sterileinheit und dem zweiten Verbindungsbereich und der Koppeleinheit mit dem ersten Verbindungsbereich detektiert hat.

Zusätzlich kann mit Hilfe des Koppelsensors einfach detektiert werden, ob zumindest die Sterileinheit korrekt mit der Sterilschleuse verbunden ist, sodass dann davon ausgegangen werden kann, dass die Sterileinheit korrekt mit der Sterilschleuse und über die Sterilschleuse korrekt mit der Koppeleinheit des Manipulatorarms verbunden ist. Dadurch ist ein gefahrloser Antrieb der angetriebenen Elemente durch die Antriebselemente möglich.

Das chirurgische Instrument umfasst vorzugsweise mindestens einen in eine Körperöffnung eines Patienten einführbaren Endeffektor, wie eine Klemme, eine Schere, einen Greifer, einen Nadelhalter, einen Mikrodissektor, eine Klammervorrichtung, eine Heftvorrichtung, eine Spül- und/oder Absaugvorrichtung, eine Schneidklinge, eine Kauterisationssonde, einen Katheter und/oder eine Saugdüse. Dadurch kann das chirurgische Instrument wahlweise unterschiedliche Endeffektoren haben, die für gängige minimal-invasive Eingriffe, insbesondere bei der laparoskopischen Chirurgie, eingesetzt werden können. Es können jedoch auch andere chirurgische Instrumente zusätzlich oder alternativ eingesetzt werden. Insbesondere kann das chirurgische Instrument auch ein optisches chirurgisches Instrument, wie ein Endoskop, sein, welches dann weitere optische und elektrische Übertragungsmittel oder Schnittstellen, wie z. B. elektrische Kontakte zur Kamerasteuerung oder zur Bilddatenübertragung optischer Lichtleitfaserverbindungen, insbesondere zur Beleuchtung, aufweist.

Ein zweiter Aspekt betrifft eine Anordnung zur robotergestützten Chirurgie, insbesondere zu einer telerobotergestützten Prozedur innerhalb eines sterilen Feldes mit Hilfe eines sterilen chirurgischen Instruments. Diese Anordnung umfasst mindestens eine Vorrichtung nach Anspruch 1 oder nach einer zuvor angegebenen Weiterbildung, eine Anzeigeeinheit, die mindestens ein Bild des Operationsgebiets, in dem sich der Endeffektor des chirurgischen Instruments befinden kann, in Echtzeit, vorzugsweise als Bildfolge, ausgibt und mindestens eine Einrichtung zur Eingabe mindestens eines Eingabekommandos. Die Anordnung hat ferner eine Steuereinheit, die den Manipulatorarm und die über die Sterilschleuse mit der Koppeleinheit des Manipulatorarms verbundene Sterileinheit abhängig von dem Eingabekommando mit Hilfe mindestens einer Antriebseinheit positioniert. Dadurch ist eine einfache Steuerung des Manipulatorarms zum Positionieren der Sterileinheit und/oder eine Bedienhandlung zum Betätigen der Sterileinheit einfach möglich. Vorzugsweise hat die Eingabeeinrichtung ein von einer Bedienperson, wie einem Chirurgen, betätigbares Betätigungselement, wobei die Eingabeeinrichtung eine Raumpositionsänderung des Betätigungselements erfasst und ein der erfassten Raumpositionsänderung entsprechendes Eingabekommando erzeugt. Die Steuereinheit erzeugt abhängig vom Eingabekommando mindestens ein Steuerkommando, durch das dieselbe oder eine maßstäblich verkleinerte Raumpositionsänderung zumindest eines Endes der Sterileinheit und/oder des chirurgischen Instruments, an dessen distalen Ende die Sterileinheit angeordnet ist, und/oder durch das eine Betätigung oder eine untersetzte Betätigung des chirurgischen Instruments, an dessen distalen Ende die Sterileinheit angeordnet ist, bewirkt wird. Dadurch ist eine einfache Positionierung und/oder Betätigung des chirurgischen Instruments durch eine entfernt vom Patienten im Operationssaal oder außerhalb des Operationssaals befindliche Bedienperson einfach möglich. Als Ausgabe eines Bildes in Echtzeit wird die unmittelbare Ausgabe eines mit Hilfe einer Bilderfassungseinheit erfassten Bildes vorzugsweise als Videosequenz ohne die bei der Bildverarbeitung entstehenden Verzögerungen hinausgehende Verzögerungen.

Ferner ist es vorteilhaft, wenn die Anordnung mehrere Vorrichtungen zur robotergestützten Chirurgie nach Anspruch 1 oder nach einer angegebenen Weiterbildung hat. Die Eingabeeinrichtung hat vorzugsweise mindestens zwei von einer Bedienperson betätigbare Betätigungselemente, wobei die Eingabeeinrichtung eine Raumpositionsänderung jedes Betätigungselements erfasst und jeweils ein der erfassten Raumpositionsänderung entsprechendes Eingabekommando erzeugt. Die Steuereinheit erzeugt abhängig von jedem Eingabekommando jeweils mindestens ein Steuerkommando, durch das dieselbe oder eine maßstäblich veränderte Raumpositionsänderung zumindest eines Endes eines chirurgischen Instruments, an dessen distalen Ende die Sterileinheit angeordnet ist, der dem jeweiligen Betätigungselement zum Zeitpunkt der Betätigung zugeordneten Vorrichtung zur robotergestützten Chirurgie und/oder durch das eine Betätigung oder eine skalierte Betätigung dieses chirurgischen Instruments bewirkt wird. Dadurch kann die Operation mit mehreren Instrumenten durchgeführt werden, die sich gleichzeitig im Operationsfeld befinden bzw. die bei laparoskopischen Eingriffen gleichzeitig in dem Bauchraum des Patienten befinden.

Die Sterilschleuse kann zwei Schleusenklappen und die Sterileinheit kann zwei Sterilklappen haben.

Die Sterilschleuse ist bei allen beschriebenen Ausführungsformen kein Bestandteil der Funktionskette zur Übertragung von elektrischer Energie, von elektrischen oder optischen Signalen und/oder mechanischer Energie zwischen dem Manipulatorarm und der Sterileinheit. Vielmehr kann die Sterilschleuse ein festes Formteil und ein zumindest die Schleusenklappe umfassendes Schleusenklappensystem umfassen, das nicht sterile Antriebselemente der Koppeleinheit so abgeschirmt, dass dieses und die gesamte Koppeleinheit nach Anbringung der sterilen Abdeckung mit der Sterilschleuse gegenüber der sterilen Umgebung steril abgedeckt sind. Der Öffnungsmechanismus des Schleusenklappensystems ist dabei vorzugsweise konstruktiv so ausgeführt, dass dieser nicht durch versehentliche Betätigung von außen geöffnet werden kann. Weiterhin können auch die angetriebenen Elemente durch ein steriles Gehäuse der Sterileinheit und insbesondere durch die mindestens eine Sterilklappe der Sterileinheit steril abgeschirmt werden.

Die Antriebselemente und die angetriebenen Elemente sind vorzugsweise derart ausgeführt, dass ein laparoskopisches chirurgisches Instrument in insgesamt vier Freiheitsgraden bewegt werden kann, nämlich:
1. Rotation des Instrumentenschaftes
2. Rotation der Instrumentenspitze unabhängig vom Instrumentenschaft
3. Abwinken der Instrumentenspitze gegenüber dem Instrumentenschaft
4. Betätigung des chirurgischen Instruments, insbesondere zum Erzeugen einer Relativbewegung von zwei zueinander beweglich angeordneten Elementen, wie der Greifbewegung der Instrumentenspitze oder von Scherenklingen.

Das sterile Gehäuse der Sterileinheit wird während der Verbindung mit der Sterilschleuse vorzugsweise in einen Aufnahmebereich des zweiten Verbindungsbereich hineingedrückt und durch eine mechanische Raste an der Sterilschleuse gegen unbeabsichtigtes Lösen gesichert. Die mechanische Raste erzeugt somit eine Rastverbindung zwischen der Sterilschleuse und der Sterileinheit. Zum Trennen der Sterileinheit von der Sterilschleuse wird manuell eine Entriegelungstaste betätigt, sodass die Sterileinheit vom zweiten Verbindungsbereich getrennt, vorzugsweise aus dem Aufnahmebereich des zweiten Verbindungsbereichs entnommen, werden kann.

Als proximal wird allgemein ein dem Patienten zugewandtes Ende eines beliebigen Elements angesehen. Als distal wird allgemein ein dem Patienten abgewandtes Ende eines Elements angesehen.

Weitere Merkmale und Vorteile ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische Darstellung eines Systems zur robotergestützten Chirurgie mit einem Manipulator, der vier Manipulatorarme hat, mit denen jeweils eine Sterileinheit einer Instrumenteneinheit verbindbar ist;
- Figur 2: eine Ansicht der in Figur 1 dargestellten Manipulators von vorn;
- Figur 3: eine perspektivische Darstellung eines Abschnitts eines Manipulatorarms mit einer Koppeleinheit zum Koppeln des Manipulatorarms mit einer eine Sterileinheit umfassenden Instrumenteneinheit, einer mit der Koppeleinheit gekoppelten Sterilschleuse und mit einer mit der Sterilschleuse gekoppelten Sterileinheit der Instrumenteneinheit;
- Figur 4: eine weitere perspektivische Darstellung der Anordnung nach Figur 3;
- Figur 5: eine Anordnung zum Verbinden der in einem sterilen Bereich einer angeordneten Instrumenteneinheit mit der nicht sterilen Koppeleinheit eines Manipulatorarms;
- Figur 6: eine schematische Darstellung der Koppeleinheit des Manipulatorarms;
- Figur 7: die Koppeleinheit nach Figur 6 mit ausgeblendeter Gehäuseoberseite;
- Figur 8: die Koppeleinheit nach den Figuren 6 und 7 ohne das obere Gehäusesegment;
- Figur 9: einen Längsschnitt der Koppeleinheit nach den Figuren 6 bis 8;
- Figur 10: eine perspektivische Darstellung der Sterilschleuse mit geschlossenen und verriegelten Sterilklappen;
- Figur 11: eine perspektivische Darstellung der Instrumenteneinheit mit geöffneten Sterilklappen der Sterileinheit;
- Figur 12: eine perspektivische Darstellung der Instrumenteneinheit nach Figur 11 mit geschlossenen Sterilklappen;
- Figur 13: die Instrumenteneinheit nach den Figuren 11 und 12 mit entfernter Bodenplatte;
- Figur 14: eine Schnittdarstellung eines Ausschnitts der Instrumenteneinheit nach Figur 13 mit mehreren mit Hilfe von Antriebselementen der Koppeleinheit antreibbaren Elementen;
- Figur 15: eine Draufsicht auf eine Anordnung der Koppeleinheit, Sterilschleuse und Instrumenteneinheit;
- Figur 16: eine Schnittdarstellung der Anordnung nach Figur 15 entlang der Schnittlinie O-O in einer ersten Position zum Verbinden der Instrumenteneinheit mit der mit der Koppeleinheit gekoppelten Sterilschleuse;
- Figur 17: eine Schnittdarstellung der Anordnung nach Figur 15 entlang der Schnittlinie O-O in einer zweiten Position zum Verbinden der Instrumenteneinheit mit der mit der Koppeleinheit gekoppelten Sterilschleuse;
- Figur 18: eine Schnittdarstellung der Anordnung nach Figur 15 entlang der Schnittlinie O-O in einer dritten Position zum Verbinden der Instrumenteneinheit mit der mit der Koppeleinheit gekoppelten Sterilschleuse;
- Figur 19: eine geschnittene Darstellung der Instrumenteneinheit, Sterilschleuse und Koppeleinheit im verbundenen Zustand;
- Figur 20: einen Ausschnitt einer Instrumenteneinheit gemäß einer zweiten Ausführungsform;
- Figur 21: eine sterile Abdeckung mit einer Sterilschleuse gemäß einer zweiten Ausführungsform;
- Figur 22: einen Ausschnitt einer Instrumenteneinheit gemäß einer dritten Ausführungsform; und
- Figur 23: eine sterile Abdeckung mit einer Sterilschleuse gemäß einer dritten Ausführungsform.

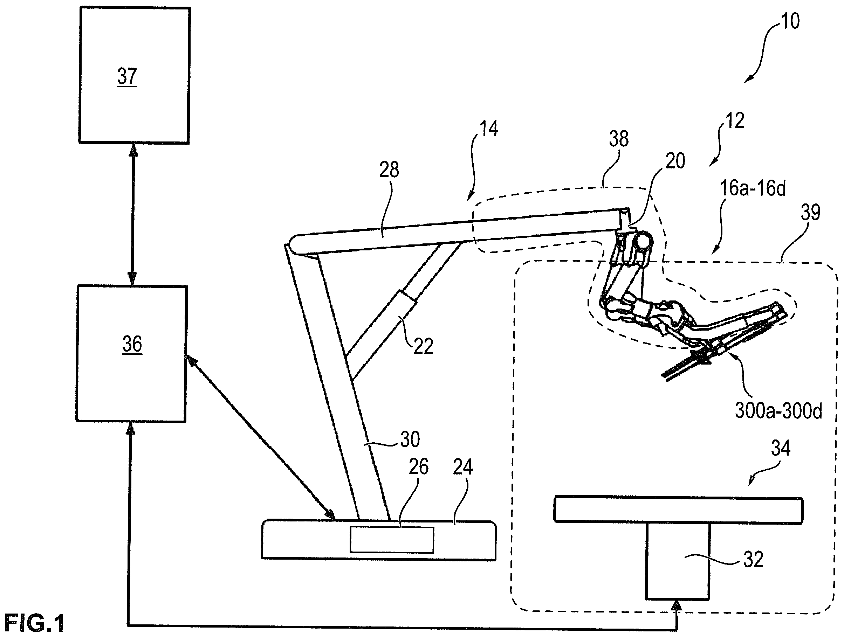

Figur 1 zeigt eine schematische Darstellung eines Systems 10 zur robotergestützten Chirurgie mit einem Manipulator 12, der ein Stativ 14 und vier Manipulatorarme 16a bis 16d hat. Bei anderen Ausführungsbeispielen kann der Manipulator 12 auch mehr oder weniger Manipulatorarme 16a bis 16d haben. Jeder Manipulatorarm 16a bis 16d ist mit einer sterilen Instrumenteneinheit 300a bis 300d über eine Koppeleinheit des Manipulatorarms 16a bis 16d verbunden. Die Instrumenteneinheit 300a bis 300d ist steril und umfasst neben einer Sterileinheit zum Koppeln der Instrumenteneinheit 300a bis 300d mit der Koppeleinheit des Manipulatorarms 16a bis 16d ein chirurgisches Instrument, insbesondere mit einem End-Effektor, wobei der End-Effektor mit Hilfe von der Koppeleinheit des Manipulatorarms 16a bis 16d bewegt und/oder betätigt werden kann. Alternativ zum chirurgischen Instrument kann die Instrumenteneinheit 300a bis 300d auch ein optisches Instrument, insbesondere ein Endoskop, und/oder ein medizinisches Gerät, insbesondere zum Applizieren eines Medikaments, zum Abgeben einer Spülflüssigkeit und/oder zum Absaugen von Spülflüssigkeit und/der Sekret, umfassen.

Das Stativ 14 hat ein auf dem Fußboden eines Operationssaals stehenden Stativfuß 24. Die Manipulatorarme 16a bis 16d sind mit einem Stativkopf 20 des Stativs 14 verbunden. Bei anderen Ausführungsformen kann das Stativ auch ein Deckenstativ sein.

Die Position des Stativkopfs 20 ist mit Hilfe einer ersten Antriebseinheit 22 und mit einer in dem Stativfuß 24 angeordneten zweiten Antriebseinheit 26 einstellbar. Mit Hilfe der Antriebseinheit 22 sind Stativarme 28, 30 relativ zueinander bewegbar. Mit Hilfe der Antriebseinheit 26 kann die Neigung des Stativarms 30 relativ zur Aufstellfläche des Stativfußes 24 geändert und/oder der Stativarm 30 um eine senkrechte Drehachse gedreht werden. Im Allgemeinen erfolgt die Positionierung des Stativkopfs 20 vor einer Operation eines Patienten. Während der Operation bleibt die Lage des Stativkopfs 20 zu der Säule 32 eines Operationstischs 34 üblicherweise unverändert. Der Manipulator 12 wird mit Hilfe einer Steuereinheit 36 gesteuert. Die Steuereinheit 36 ist über eine Daten- und/oder Steuerleitung mit einer Ein- und Ausgabeeinheit 37 verbunden, die insbesondere ein Bild des Operationsfelds an eine Bedienperson in Echtzeit mit Hilfe mindestens einer Anzeigeeinheit ausgibt. Die Bedienperson macht Bedieneingaben, durch die die Instrumenteneinheiten 300a bis 300d während der Operation des Patienten positioniert und betätigt werden. Die Ein- und Ausgabeeinheit 37 dient somit als Mensch-Maschinen-Schnittstelle.

Die Steuereinheit 36 ist ferner über eine Steuer- und/oder Datenverbindung mit einer nicht dargestellten Steuereinheit des Operationstischs 34 verbunden. Über diese Steuer- und/oder Datenverbindung wird sichergestellt, dass die Lage der Patientenlagerfläche oder von Segmenten der Patientenlagerfläche des Operationstischs 34 nur dann verändert werden kann, wenn dies aufgrund der Positionierung der Instrumenteneinheiten 300a bis 300d für einen zu operierenden Patienten gefahrlos möglich ist.

Der Operationstisch 34 sowie die Instrumenteneinheiten 300a bis 300d sind in einem sterilen Operationsbereich 39 angeordnet. Die Manipulatorarme 16a bis 16d und das Stativ 14 sind nicht steril. Die in dem sterilen Operationsbereich 39 ragenden Bereiche des Manipulators 12, d.h. die Manipulatorarme 16a bis 16d, der Stativkopf 20 und ein Teil des Stativarms 28, sind in einer mit Hilfe der Strichlinie angedeuteten sterilen flexiblen Hülle 38, wie einer Sterilfolie, steril verpackt, sodass sie gefahrlos im sterilen Operationsbereich 39 angeordnet werden können. Die Ein- und Ausgabeeinheit 37 ist außerhalb des sterilen Bereichs 39 angeordnet und muss deshalb nicht steril verpackt werden.

Bei einer Vielzahl von Operationen müssen die Instrumenteneinheiten 300a bis 300d während der Operation aufgrund des Operationsverlaufs mehrfach gewechselt werden. Somit muss zwischen dem Manipulatorarm 16a bis 16d und der Instrumenteneinheit 300a bis 300d eine sterile Schnittstelle vorgesehen werden, die sicherstellt, dass die nicht sterilen Teile der Koppeleinheit des Manipulatorarms 16a bis 16d auch nach dem Trennen der Instrumenteneinheit 300a bis 300d steril abgedeckt sind. Darüber hinaus müssen durch einen Kontakt der sterilen Elemente der Koppeleinheit des Manipulatorarms 16a bis 16d kontaminierte Elemente der Instrumenteneinheit 300a bis 300d nach dem Trennen der Instrumenteneinheit 300a bis 300d vom Manipulatorarm 16a bis 16d steril abgedeckt werden, damit die Instrumenteneinheit 300a bis 300d im sterilen Bereich 39 abgelegt werden kann, ohne weitere Elemente im sterilen Bereich 39 zu kontaminieren. Dazu wird eine Sterilschleuse zwischen der Koppeleinheit des Manipulatorarms 16a bis 16d und der Instrumenteneinheit 300a bis 300d vorgesehen, die mindestens eine Schleusenklappe hat, die geschlossen ist, wenn keine Instrumenteneinheit 300a bis 300d mit der Sterilschleuse verbunden ist, sodass dann die nicht sterile Koppeleinheit vom sterilen Bereich 39 mit Hilfe der flexiblen sterilen Hülle 38 und der in diese integrierten Sterilschleuse vom sterilen Bereich 39 abgeschirmt ist.

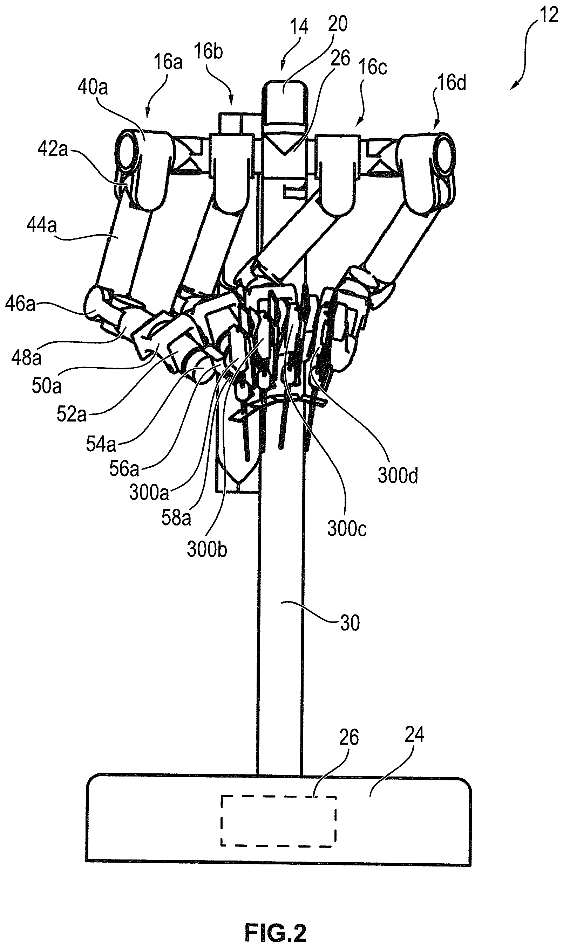

In Figur 2 ist eine Frontansicht des Manipulators 12 nach Figur 1 gezeigt. Die Manipulatorarme 16a bis 16d des Manipulators 12 haben jeweils mehrere Segmente 40a bis 58a, die mit Hilfe integrierter Antriebseinheiten relativ zueinander bewegbar sind, so dass die Instrumenteneinheiten 300a bis 300d ohne Kollision exakt positioniert werden können. Die sterilen Hüllen 38 zum Abschirmen eines Abschnitts der Manipulatorarme 16a bis 16d sind in Figur 2 nicht dargestellt. Die Segmente des Manipulatorarms 16a sind mit den Bezugszeichen 40a bis 58a bezeichnet. Die weiteren Manipulatorarme 16b bis 16d haben den gleichen Aufbau und haben die in Figur 2 aus Übersichtlichkeitsgründen nicht bezeichneten Segmente 40b bis 58b, 40c bis 58c und 40d bis 58d. Dieselben Elemente der Manipulatorarme 16a bis 16d sind mit derselben Bezugszeichenziffer und mit dem zusätzlichen Buchstaben zur Unterscheidung der Manipulatorarme 16a bis 16d bezeichnet. Die in der nachfolgenden Beschreibung gemachten Ausführungen beziehen sich auf den Manipulatorarm 16a und die Instrumenteneinheit 300a, die im Folgenden mit Manipulatorarm 16 und Instrumenteneinheit 300 bezeichnet sind. Die Segmente 40a bis 58a des Manipulatorarms 16a sind im Folgenden als Segmente 40 bis 58 bezeichnet. Die Ausführungen gelten jedoch in gleicher Weise für die gleich aufgebauten Manipulatorarme 16b bis 16d und die Instrumenteneinheiten 300b bis 300d. Elemente mit gleichem Aufbau und/oder gleicher Funktion sind mit denselben Bezugszeichen bezeichnet.

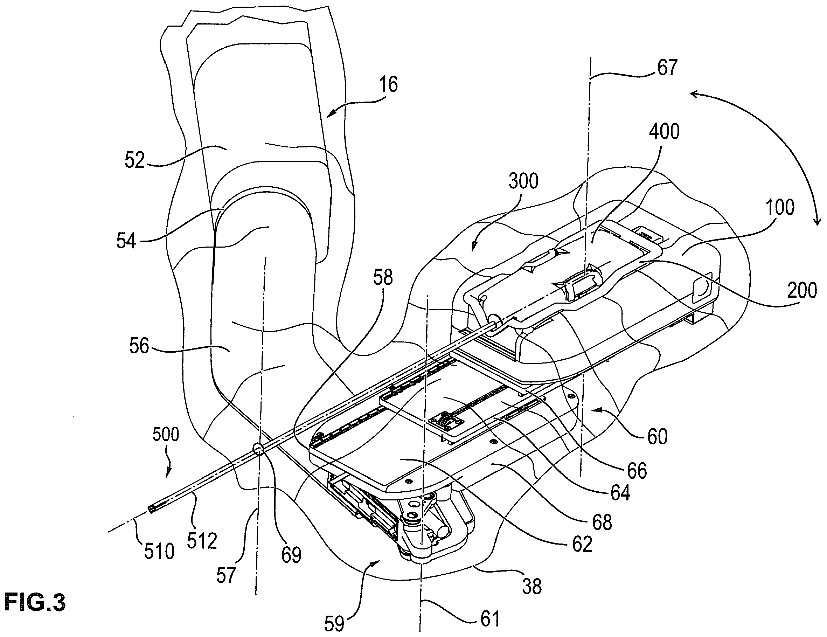

Figur 3 zeigt eine perspektivische Darstellung eines Abschnitts des Manipulatorarms 16 mit einer Koppeleinheit 100 zum Koppeln des Manipulatorarms 16 mit der eine Sterileinheit 400 umfassenden Instrumenteneinheit 300. Hierzu ist die Koppeleinheit 100 mit einer in die sterile Hülle 38 integrierten Sterilschleuse 200 verbunden. Die Sterilschleuse 200 ist sowohl mit der Koppeleinheit 100 als auch mit der Sterileinheit 400 koppelbar und wieder trennbar. In Figur 3 ist die Sterilschleuse 200 sowohl mit der Koppeleinheit 100 als auch mit der Sterileinheit 400 gekoppelt dargestellt. Die Koppeleinheit 100 ist am distalen Ende der Teleskopanordnung 60 angeordnet.

Die Teleskopanordnung 60 hat zueinander verschiebbare Abschnitte 62, 64, 66 und ist in Figur 3 in einem ausgefahrenen Zustand dargestellt. Die Abschnitte 62, 64, 66 der Teleskopanordnung 60 können mit Hilfe einer Antriebseinheit 68 eingefahren und ausgefahren werden, sodass ein chirurgisches Instrument 500 der Instrumenteneinheit 300 entlang der Längsachse 510 des Instrumentenschafts 512 zusammen mit der Koppeleinheit 100, der Sterilschleuse 200 und der Sterileinheit 400 bewegt werden kann. Mit Hilfe einer in das Segment 52 integrierten Antriebseinheit kann das Segment 54 zusammen mit dem als Gelenkarm ausgeführten Segment 56 um die Drehachse 57 gedreht werden. Das Segment 58 ist über ein Koppelgetriebe 59 mit dem Segment 56 verbunden, sodass das Segment 58 nach Aktivierung einer mit dem Koppelgetriebe 59 verbundenen Antriebseinheit um die Drehachse 61 geschwenkt werden kann. Ferner ist die Koppeleinheit 100 über ein in Figur 3 nicht sichtbares Koppelgetriebe gegenüber dem Segment 66 um die Drehachse 67 drehbar angeordnet. Auch dieses Koppelgetriebe ist über eine mit diesem Koppelgetriebe verbundenen Antriebseinheit antreibbar, sodass bei Aktivierung dieser Antriebseinheit die Koppeleinheit 100 um die Drehachse 67 gedreht wird. Dabei werden die Antriebseinheiten der Koppelgetriebe derart angesteuert, dass die Längsachse 510 des Instrumentenschafts 512 bei einer Bewegung des Manipulatorarms 16 und dessen Segmente um einen im Raum ortsfesten Pivot-Punkt 69 geschwenkt werden, durch den die Längsachse 510 des bei einer Operation vorzugsweise durch einen Trokar in einen Patienten eingeführten Instrumentenschaft 512 wird dann um den Pivot-Punkt 69 gedreht, sodass sichergestellt ist, dass durch eine Bewegung des Instruments 500 nur eine geringe Beanspruchung des Patienten an der Eintrittsstelle des Instruments 500 in dem Patienten erfolgt und insbesondere eine Verletzung des Patienten an der Eintrittsstelle des Instrumentenschafts 512 verhindert wird.

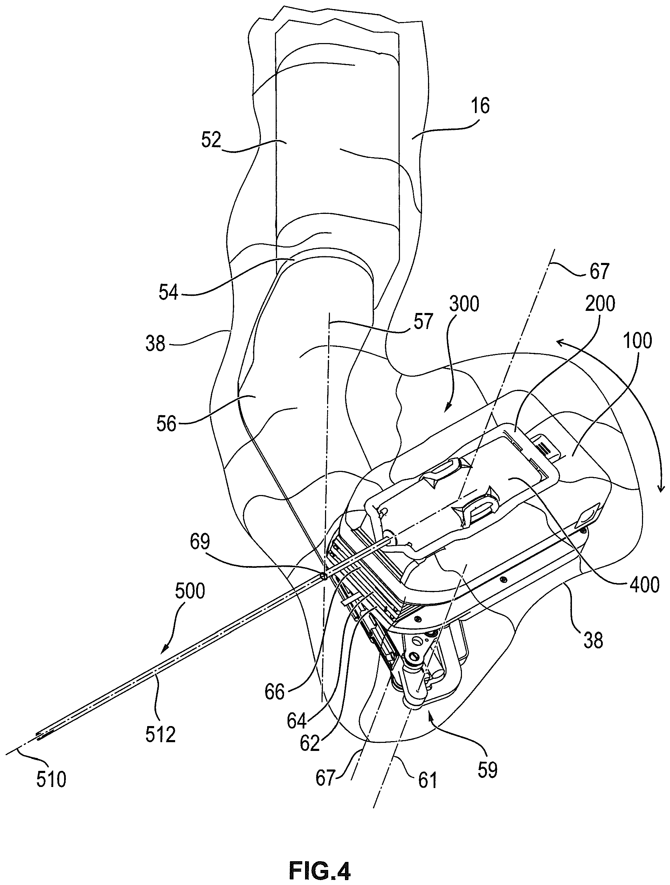

In Figur 4 ist eine weitere perspektivische Darstellung der Anordnung nach Figur 3 gezeigt, wobei die Abschnitte 62, 64, 66 der Teleskopanordnung 60 im Unterschied zu Figur 3 in einem eingefahrenen Zustand dargestellt sind, wodurch die Instrumenteneinheit 300 in Richtung der Längsachse 510 des Instrumentenschafts 512 zum proximalen Ende des chirurgischen Instruments 500 hin verschoben worden ist. Somit ist durch das Einfahren der Teleskopanordnung 60 die Instrumenteneinheit 300 in Richtung des proximalen Endes des Instruments 500 entlang der Längsachse 510 des Instruments 500 verschoben worden. Dabei ist jedoch die Lage des Pivot-Punkts 69 unverändert geblieben. Auch bei einer Drehung der Segmente 56, 58, 60 um die Drehachse 57 wird der Pivot-Punkt 69 durch eine entsprechende Ansteuerung der Antriebseinheiten der Koppelgetriebe 59 in seiner Raumposition unverändert gelassen, in dem eine entsprechende Drehung des Segments 60 um die Drehachse 61 und der Koppeleinheit 100 um die Drehachse 67 erfolgt. Ferner verläuft eine durch einen entsprechenden Antrieb der Koppelgetriebe erzeugte virtuelle Drehachse (nicht dargestellt), die parallel zu den Drehachsen 61, 67 und orthogonal zu der Drehachse 57 verläuft, durch den Pivot-Punkt 69.

In dem Pivot-Punkt 69 schneiden sich die Drehachse 57 des als Gelenkarm ausgebildeten Segments 56 und die Längsachse 510 des Instruments 500. Der Pivot-Punkt 69 wird auch als Pivotalpunkt bezeichnet.

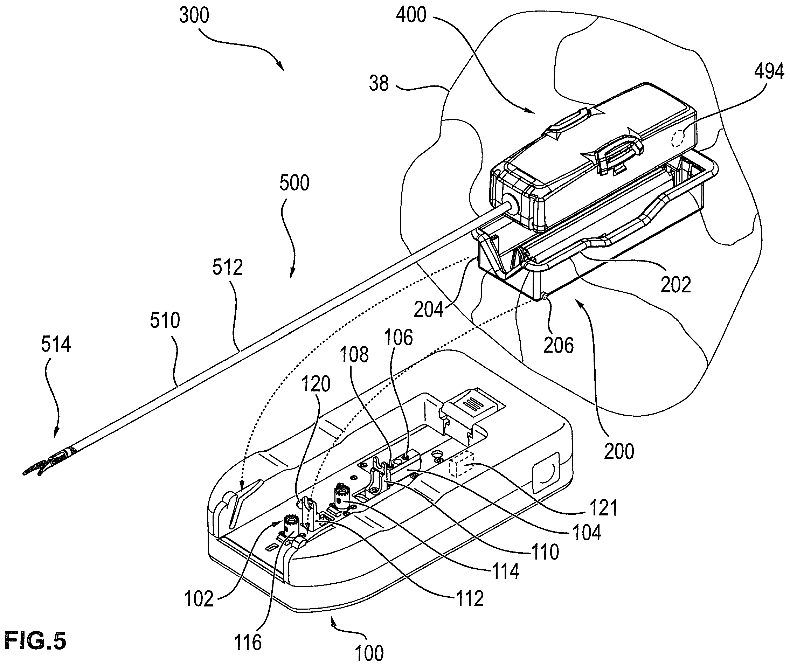

Figur 5 zeigt die Koppeleinheit 100, die Sterilschleuse 200 sowie die Instrumenteneinheit 300 mit der Sterileinheit 400 und dem chirurgischen Instrument 500, das einen Endeffektor 514 hat, vor dem Zusammenfügen der Sterilschleuse 200 mit der Koppeleinheit 100 und vor dem nachfolgenden Zusammenfügen der Sterileinheit 400 mit der Sterilschleuse 200. Die als Sterilfolie ausgeführte flexible sterile Hülle 38 ist an einem umlaufenden Anschlussrand 202 der Sterilschleuse 200 mit dieser über eine geeignete Verbindung, wie eine Klemm-, Klebe- und/oder Schweißverbindung, fest verbunden, so dass die Sterilfolie 38 zusammen mit der Sterilschleuse 200 eine geschlossene sterile Abdeckung um die vom sterilen Bereich 39 abzuschirmenden nicht sterilen Elemente 16, 100 bildet, wie dies auch in den Figuren 1, 3 und 4 gezeigt ist. Zur besseren Darstellung ist in Figur 5 nur ein Ausschnitt der Sterilfolie 38 um die Sterilschleuse 200 herum dargestellt. In den nachfolgenden Figuren ist die Sterilfolie 38 teilweise nicht dargestellt.

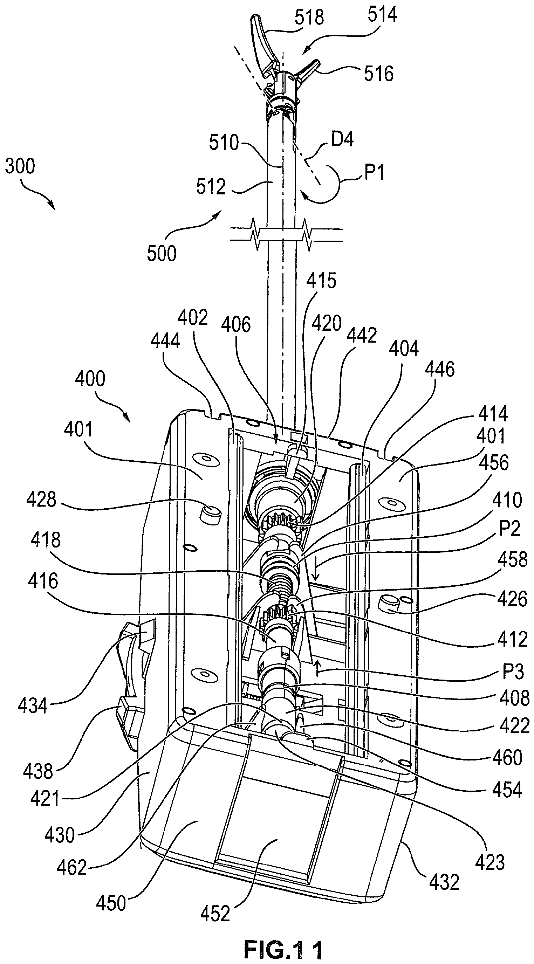

Zur Kopplung der Sterileinheit 400 mit der Koppeleinheit 100 ist die Sterilschleuse 200 zwischen der Sterileinheit 400 und er Koppeleinheit 100 angeordnet und ermöglicht im gekoppelten Zustand der Sterileinheit 400 mit der Koppeleinheit 100 eine direkte Kopplung eines ersten Übertragungsmittels 102 der Koppeleinheit 100 und eines zweiten Übertragungsmittels der Sterileinheit 400. Das zweite Übertragungsmittel ist in Figur 11 mit dem Bezugszeichen 406 bezeichnet.

Mit Hilfe des ersten Übertragungsmittels 102 wird im vorliegenden Ausführungsbeispiel sowohl mechanische Energie als auch elektrische Energie zwischen der Koppeleinheit 100 und der Sterileinheit 400 übertragen. Hierzu hat das erste Übertragungsmittel 102 der Koppeleinheit 100 mindestens vier mechanische Antriebselemente 110 bis 116 und das zweite Übertragungsmittel 406 der Sterileinheit 400 vier zu den Antriebselementen 110 bis 116 in Figur 11 dargestellte komplementäre angetriebene Elemente 412 bis 418. Weiterhin hat das erste Übertragungsmittel 102 ein elektrisches Übertragungselement 104 mit zwei elektrischen Kontakten 106, 108 und das zweite Übertragungsmittel 406 ein zum elektrischen Übertragungselement 104 des ersten Übertragungsmittels 102 komplementäres elektrisches Übertragungselement. Das komplementäre elektrische Übertragungselement umfasst zwei in Figur 11 dargestellte elektrische Kontakte 422, 423.

Bei anderen Ausführungsbeispielen können die ersten und zweiten Übertragungsmittel auch mehr oder weniger Antriebselemente, angetrieben Elemente und elektrische Übertragungselemente umfassen, die mechanische und/oder elektrische Energie durch direkte Kopplung übertragen. Als direkte Kopplung wird dabei eine Kopplung der Übertragungsmittel angesehen, bei der keine weiteren Übertragungselemente zwischen den ersten Übertragungsmitteln und den zweiten Übertragungsmitteln für eine Übertragung von mechanischer und/oder elektrischer Energie und/oder optischen Strahlen vorgesehen sind, wobei insbesondere keine elektrischen, mechanischen oder optischen Übertragungselemente in einer zwischen der Koppeleinheit 100 und der Sterileinheit 400 angeordneten Sterilbarriere, wie der Sterilschleuse 200, vorgesehen sind. Die Koppeleinheit 100 hat ferner eine RFID-Lese-und-Schreibeinheit 121, mit deren Hilfe ein RFID-Transponder 494 der Sterileinheit 400 lesbar und/oder schreibbar ist.

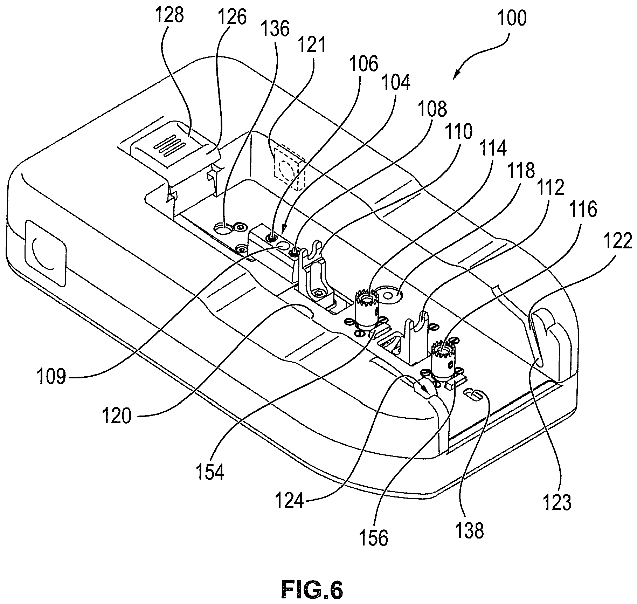

Figur 6 zeigt eine schematische perspektivische Darstellung der Koppeleinheit 100 des Manipulatorarms 16. Das erste Übertragungsmittel 102 der Koppeleinheit 100 hat ein elektrisches Übertragungselement 104 mit zwei elektrischen Kontakten 106, 108, ein optisches Übertragungsmittel 109 zur Übertragung von Licht und/oder optischen Signalen, ein erstes translatorisches Antriebselement 110 und ein zweites translatorisches Antriebselement 112 jeweils zur Übertragung einer translatorischen Bewegung sowie ein erstes rotatorisches Antriebselement 114 und ein zweites rotatorisches Antriebselement 116 zur Übertragung einer Rotationsbewegung. Das erste und das zweite translatorische Antriebselement 110, 112 sind jeweils als Linearhubgabel und das erste und das zweite rotatorische Antriebselement 114, 116 sind als Antriebsritzel mit stirnseitiger Zahnung ausgebildet. Ferner hat die Koppeleinheit 100 einen in einer Vertiefung angeordneten ersten Koppelsensor 118, der ein durch einen aus der Sterileinheit 400 vorstehenden ersten Detektionsstift gebildetes erstes Detektionselement detektiert, wenn die Sterilschleuse 200 korrekt mit der Koppeleinheit 100 und die Sterileinheit 400 korrekt mit der Sterilschleuse 200 gekoppelt ist. In diesem Fall ragt ein erster Detektionsstift der Sterileinheit 400 in die Vertiefung, in der der erste Koppelsensor 118 angeordnet ist, so dass dieser das Vorhandensein des als erstes Detektionselement dienenden ersten Detektionsstifts detektiert. Der erste Detektionsstift ist in Figur 11 gezeigt und dort mit dem Bezugszeichen 426 bezeichnet.

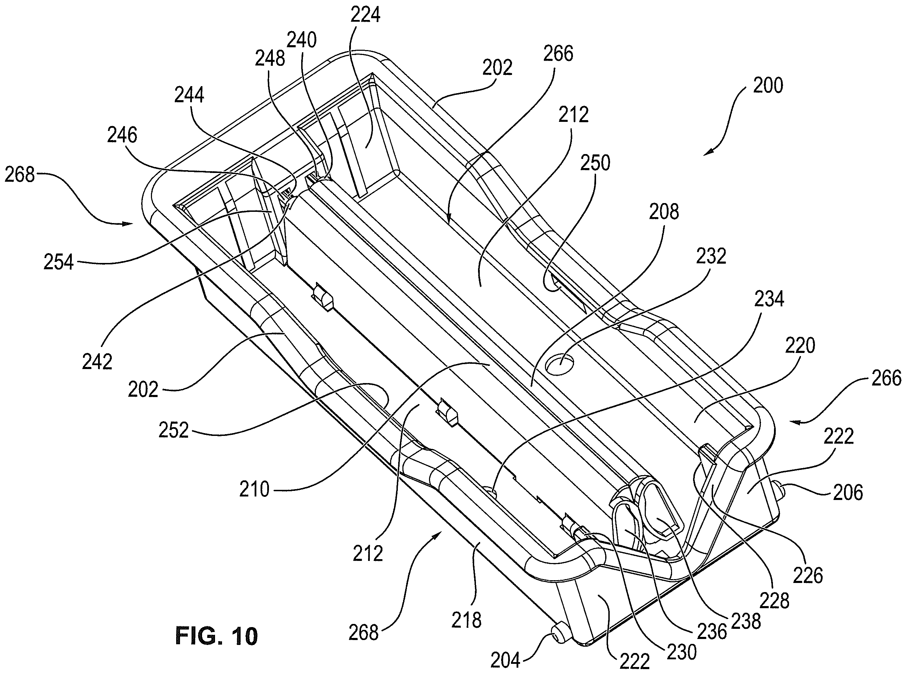

Die Koppeleinheit 100 hat einen zweiten Koppelsensor 120, der seitlich neben den Antriebselementen 112, 114 in einer weiteren Vertiefung angeordnet ist, wie dies in Figur 5 deutlicher zu sehen ist. Der zweite Koppelsensor 120 detektiert ein durch einen zweiten Detektionsstift der Sterileinheit 400 gebildetes zweites Detektionselement, wenn sowohl die Koppeleinheit 100 korrekt mit der Sterilschleuse 200 als auch die Sterilschleuse 200 korrekt mit der Sterileinheit 400 gekoppelt sind. Der zweite Detektionsstift ist in Figur 11 gezeigt und dort mit dem Bezugszeichen 428 bezeichnet. Somit wird mit Hilfe der Koppelsensoren 118, 120 sicher festgestellt, ob die Sterileinheit 400 korrekt mit der Koppeleinheit 100 gekoppelt ist, so dass eine direkte Übertragung zwischen dem ersten Übertragungsmittel 102 der Koppeleinheit 100 und dem zweiten Übertragungsmittel der Sterileinheit 400 möglich ist. Zum Verbinden der Koppeleinheit 100 mit der Sterilschleuse 200 hat die Koppeleinheit 100 einander gegenüberliegende Führungsnuten 122, 124, in die Führungsstifte 204, 206 der Sterilschleuse 200 eingeführt werden, bis sie das vordere Ende 123, 125 der jeweiligen Führungsnut 122, 124 erreicht haben, wie dies in Figur 10 gezeigt ist. Die Führungsstifte 204, 206 ragen an einem ersten Ende der Sterilschleuse 200 auf gegenüberliegenden Seiten nach außen, wie dies in den Figuren 5 und 10 zu sehen ist. Anschließend wird das gegenüberliegende zweite Ende der Sterilschleuse 200 nach unten gedrückt, so dass die Sterilschleuse 200 um eine durch die Führungsstifte 204, 206 verlaufende Drehachse gedreht wird, bis eine Rastnase 126 eines Rastelements 128 in einen komplementären Rastbereich der Sterilschleuse 200 eingreift.

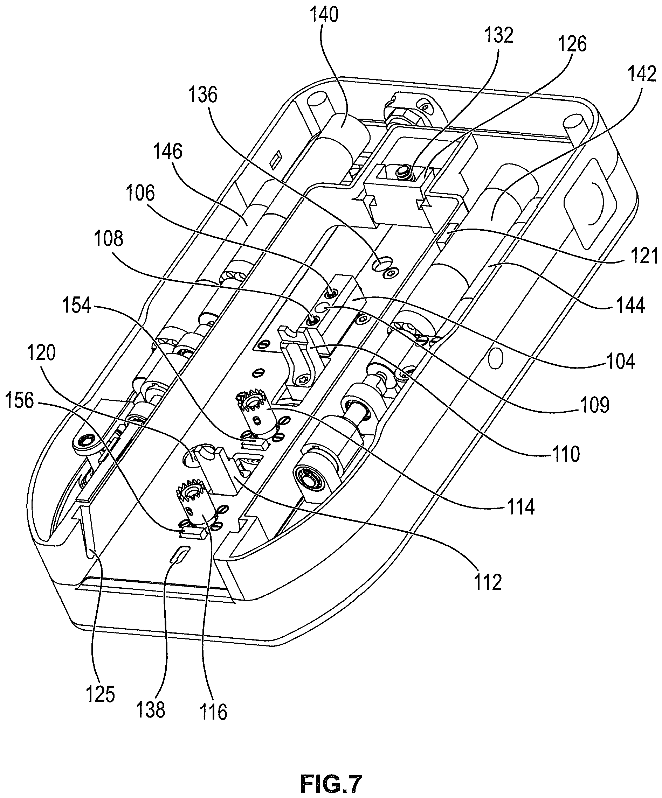

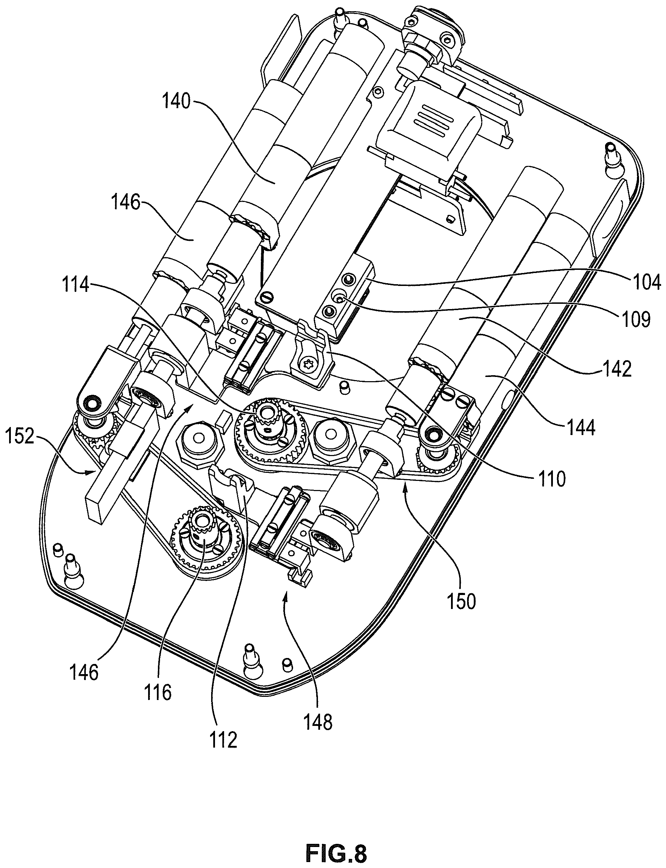

Figur 7 zeigt eine Koppeleinheit 100 nach Figur 6 mit ausgeblendeter Gehäuseoberseite und Figur 8 die Koppeleinheit 100 nach den Figuren 6 und 7 ohne das obere Gehäusesegment. Die Koppeleinheit 100 hat insgesamt vier Antriebsmotore 140 bis 146, die jeweils als Gleichstrommotore mit Tachometer ausgeführt sind, sodass die Steuereinheit 38 jederzeit über den Drehwinkel des jeweiligen Motors Kenntnis hat und dies bei der weiteren Ansteuerung berücksichtigen kann. Der erste Antriebsmotor 140 ist über ein erstes Linearkoppelgetriebe 147 mit dem ersten translatorischen Antriebselement 110 gekoppelt, das bei einer Aktivierung des Antriebsmotors 140 durch die Steuereinheit 38 eine translatorische Antriebsbewegung ausführt. Der zweite Antriebsmotor 142 ist über ein zweites Linearkoppelgetriebe 148 mit dem zweiten translatorischen Antriebselement 112 gekoppelt, sodass bei einer Antriebsbewegung des zweiten Antriebsmotors 142 das zweite translatorische Antriebselement 112 eine translatorische Antriebsbewegung ausführt. Der dritte Antriebsmotor 144 ist über eine erste Getriebestufe 150 mit dem ersten rotatorischen Antriebselement 114 gekoppelt, sodass bei einer Antriebsbewegung des dritten Antriebsmotors 144 das erste rotatorische Antriebselement 114 gedreht wird. Der vierte Antriebsmotor 146 ist über eine zweite Getriebestufe 152 mit dem zweiten rotatorischen Antriebselement 116 gekoppelt, sodass dieses bei einer Antriebsbewegung des vierten Antriebsmotors 146 eine Rotationsbewegung ausführt.

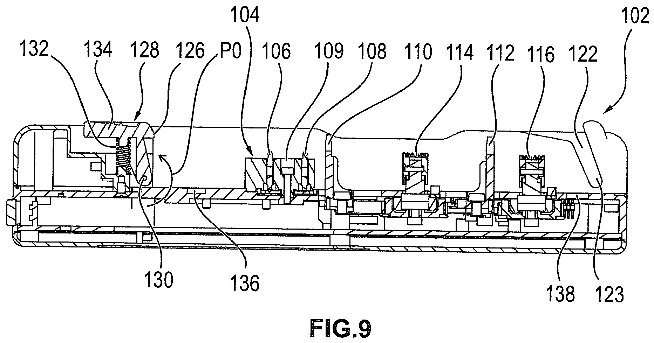

Figur 9 zeigt einen Längsschnitt durch die Koppeleinheit 100. Die Entriegelungstaste 128 ist um eine Drehachse 130 schwenkbar angeordnet und wird durch eine Feder 132 in ihrer in Figur 9 gezeigten Rastposition gehalten. Zum Lösen der Rastverbindung wird mit einem Finger auf eine Entriegelungstaste 134 des Rastelements 128 gedrückt, so dass eine Feder 132 gespannt und das Rastelement 128 zusammen mit der Rastnase 126 in Richtung des Pfeils P0 gedreht wird, so dass die Rastnase 126 außer Eingriff mit dem komplementären Rastelement der Sterilschleuse 200 gebracht wird. Dadurch kann das zuvor mit der Rastnase 126 in Eingriff stehende zweite Ende der Sterilschleuse 200 aus der Koppeleinheit 100 herausgeschwenkt werden. Nachdem dieses zweite Ende der Sterilschleuse 200 aus der Koppeleinheit 100 herausgeschwenkt worden ist, kann die Sterilschleuse 200 vollständig von der Koppeleinheit 100 getrennt werden, indem die Sterilschleuse 200 mit den mit den Führungsnuten 122, 124 in Eingriff stehenden Führungsstiften 204, 206 entlang der Führungsnuten 122, 124 aus diesem heraus gezogen wird, bis die Führungselemente 204, 206 nicht mehr in Eingriff mit den Führungsnuten 122, 124 stehen. Zwischen den Führungsnuten 122, 124 und dem Rastelement 128 ist ein durch eine entsprechende Vertiefung im Gehäuse der Koppeleinheit 100 gebildeter Aufnahmebereich vorhanden, der im vorliegenden Ausführungsbeispiel die Sterilschleuse 200 auf drei Seiten und bodenseitig zumindest teilweise umgibt.

Figur 10 zeigt eine perspektivische Ansicht der Sterilschleuse 200 mit geschlossenen Schleusenklappen 208, 210. Die Sterilschleuse 200 hat einen Boden 212, in dem zwei mit Hilfe der Schleusenklappen 208, 210 abdeckbare Öffnungen 214, 216 vorgesehen sind. Die Schleusenklappen 208, 210 sind über Scharniere schwenkbar mit dem Boden 212 verbunden. Mit Hilfe dieser Scharniere sind die Schleusenklappen 208, 210 von dem in Figur 10 gezeigten geschlossenen Zustand in den geöffneten Zustand schwenkbar. Im geöffneten Zustand der Schleusenklappen 208, 210 kann eine direkte Kopplung der Antriebselemente 110 bis 116 der Koppeleinheit 100 mit den angetriebenen Elementen der Sterileinheit 400 erfolgen. Ferner kann bei geöffneten Schleusenklappen 208, 210 eine direkte Kopplung des elektrischen Übertragungselements 104 der Koppeleinheit 100 mit dem elektrischen Übertragungselement der Sterileinheit 400 erfolgen.

Die Sterilschleuse 200 hat ferner zwei Seitenwände 218, 220, eine vordere Stirnwand 222 und eine hintere Stirnwand 224. An den Außenseiten der Seitenwände 218, 220 und den Stirnwänden 222, 224 ist der umlaufende Rand 202 ausgebildet, mit dem, wie bereits in Verbindung mit Figur 5 beschrieben, die Sterilfolie der sterilen Abdeckung 38 auf geeignete Art und Weise verbunden ist.

An der Innenseite der vorderen Stirnwand 222 sind jeweils seitlich neben einer V-förmigen Aussparung 226 der Stirnwand 222 zwei Führungs- und Entriegelungsstege 228, 230 fest angeordnet, die bei einer Verbindung der Sterilschleuse 200 mit der Sterileinheit 400 als Entriegelungselemente zum Entriegeln von Sterilklappen der Sterileinheit 400 dienen.

Im Boden 212 der Sterilschleuse 200 sind ein erstes Detektionsfenster 232 und ein zweites Detektionsfenster 234 in Form jeweils eines Durchgangslochs vorhanden, durch die die bereits erwähnten Detektionselemente 426, 428 der Sterileinheit 400 hindurchgeführt werden, so dass sie vom ersten Koppelsensor 118 und vom zweiten Koppelsensor 120 der Koppeleinheit 100 detektiert werden können.

Am vorderen und hinteren Ende der Schleusenlappen 208, 210 ist jeweils eine Führungssicke 236 bis 242 vorgesehen. Die vorderen Führungssicken 236, 238 sind ohne Funktion. In die hinteren Führungssicken 240, 242 greifen im geschlossenen Zustand der Schleusenkappen 208, 210 die Zinken 246, 248 einer Führungsgabel 244 ein. Die Führungsgabel 244 wird mit Hilfe einer Feder in ihre obere in Figur 10 gezeigte Position gedrückt und schließt durch den Eingriff ihrer Zinken 246, 248 in die Führungssicken 240, 242 die Schleusenklappen 208, 210 und hält diese in ihrer geschlossenen Position. Die Schleusenklappen 208, 210 können durch den Eingriff der Gabelzinken 246, 248 nicht auseinandergedrückt werden, so dass das nicht sterile Übertragungsmittel 102 der Koppeleinheit 100 bei geschlossenen Schleusenklappen 208, 210 sicher abgedeckt ist und die nicht sterilen Elemente der Koppeleinheit 100 sicher vom sterilen Bereich 39 abgeschirmt sind.

In den Seitenwänden 218, 220 ist jeweils eine Rastsicke 250, 252 vorgesehen, in die ein Rastelement der Sterileinheit 400 bei einer Verbindung der Sterilschleuse 200 mit der Sterileinheit 400 eingreift. An der hinteren Stirnwand 224 der Sterilschleuse 200 ist ein Führungssteg 254 vorgesehen, der bei einer Verbindung der Sterilschleuse 200 mit der Sterileinheit 400 in eine Führungsnut 452 der Sterileinheit 400 eingreift, wie dies in Figur 11 gezeigt ist.

An der Außenseite der hinteren Stirnwand 224 der Sterilschleuse 200 ist eine Rastnase ausgebildet, in die die Rastnase 126 des Rastelements 128 der Koppeleinheit 100 eingreift, wenn die Koppeleinheit 100 mit der Sterilschleuse 200 verbunden ist.

Wie aus Figur 10 ersichtlich ist, bilden die Seitenwände 218, 220, die Stirnwände 222, 224 und der Boden 218 eine Gehäusewanne, in die die Sterileinheit 400 zum Verbinden der Sterileinheit 400 mit der Koppeleinheit 100 zumindest teilweise einsetzbar ist. Die Gehäusewanne dient somit allgemein als erster Verbindungsbereich 266 der Sterilschleuse 200. Die Außenseite der Sterilschleuse 200 dient als zweiter Verbindungsbereich 268, mit dem die Sterilschleuse 200 mit der Koppeleinheit 100 verbindbar ist.

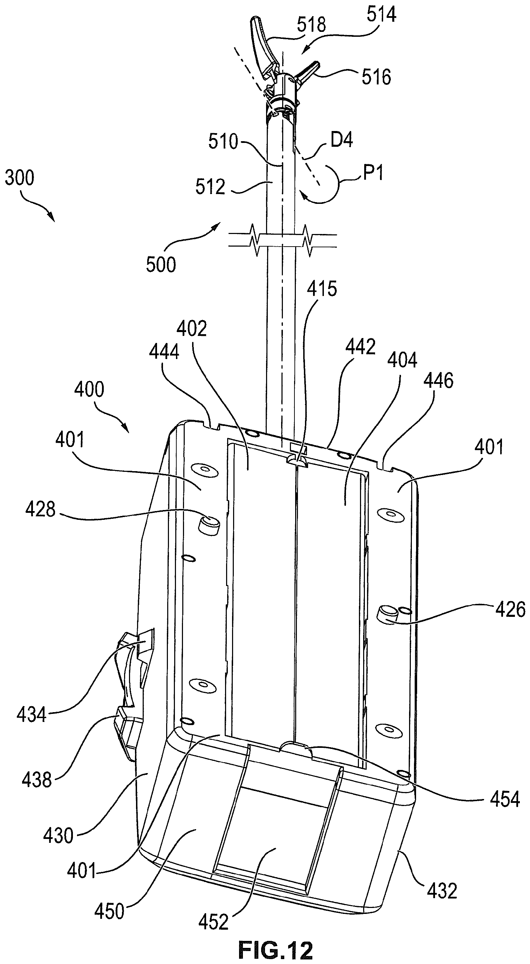

Figur 11 zeigt eine perspektivische Darstellung der Instrumenteneinheit 300 mit der Sterileinheit 400 und dem chirurgischen Instrument 500. Am proximalen Ende des drehbaren äußeren Instrumentenschafts 512 ist der abwinkelbare und drehbare Endeffektor 5124 mit betätigbaren Greifarmen 516, 518 angeordnet. Die Bewegungen des Endeffektors 514 sind mit Hilfe der Antriebselemente 110 bis 116 der Koppeleinheit 100 und der angetriebenen Elemente 408 bis 414 der Sterileinheit 400 ausführbar, wenn die Sterileinheit 400 über die Sterilschleuse 200 mit der Koppeleinheit 100 verbunden ist. Die Sterileinheit 400 hat Sterilklappen 402, 404, die in Figur 11 in einem geöffneten und in Figur 12 in einem geschlossenen Zustand gezeigt sind. Im Inneren der Sterileinheit 400 ist das zweite Übertragungsmittel angeordnet, das bei geöffneten Sterilklappen 402, 404 sichtbar und mit dem Bezugszeichen 406 bezeichnet ist. Das zweite Übertragungsmittel 406 umfasst ein bei einer Kopplung mit der Koppeleinheit mit dem ersten translatorischen Antriebselement 110 in Eingriff stehendes erstes translatorisches angetriebenes Element 408 und ein mit dem zweiten translatorischen Antriebselement 112 der Koppeleinheit 100 in Eingriff stehendes zweites translatorisches angetriebenes Element 410 jeweils zur Übertragung einer Translation. Ferner sind ein mit dem ersten rotatorischen Antriebselement 114 der Koppeleinheit 100 koppelbares erstes rotatorisches angetriebenes Element 412 sowie ein mit dem zweiten rotatorischen Antriebselement 116 der Koppeleinheit 100 in Eingriff stehendes zweites rotatorisches angetriebenes Element 414 jeweils zur Übertragung einer Rotationsbewegung vorgesehen. Bei dem mit der Koppeleinheit 400 verbundenen chirurgischen Instrument 500 wird der Endeffektor 514 um die Kippachse D4 in Pfeilrichtung P1 um bis zu 90° geschwenkt, wenn das zweite translatorische angetriebene Element 410 der Sterileinheit 400 von dem zweiten translatorischen Antriebselement 112 der Koppeleinheit 100 in Richtung des Pfeils P2 bewegt wird. Bei einer Bewegung des ersten translatorisch angetriebenen Elements 408 in Richtung des Pfeils P3 werden die Greifarme 516, 518 des Endeffektors 514 auseinanderbewegt und in entgegengesetzte Richtungen aufeinander zu bewegt. Beim Antrieb des ersten rotatorisch angetriebenen Elements 412 der Sterileinheit 400 mit Hilfe des ersten rotatorischen Antriebselements 114 der Koppeleinheit 100 kann der Endeffektor 514 unabhängig vom Instrumentenschaft 512 gedreht werden. Mit Hilfe des zweiten rotatorisch angetriebenen Elements 414 kann bei einer Kopplung und Antrieb mit dem zweiten rotatorischen Antriebselement 116 der Koppeleinheit 100 eine Rotation des Instrumentenschafts 512 um seine Längsachse 510 erfolgen, um die Lage der Kippachse D4 des Endeffektors 514 um die Drehachse 510 des äußeren Instrumentenschafts 512 zu drehen, ohne dass der Endeffektor 514 selbst mitgedreht wird.

Ferner ist eine erste Feder 416 vorgesehen, die das erste translatorische angetriebene Element 408 der Sterileinheit 400 entgegen der Pfeilrichtung des Pfeils P3 in seine Endlage drückt. Ferner ist eine zweite Feder 418 vorgesehen, die das zweite translatorisch angetriebene Element 410 der Sterileinheit 400 entgegen der Pfeilrichtung des Pfeils P2 in seine Endlage drückt. Ferner hat die Sterileinheit 400 ein Lager 420 zum drehbaren Lagern des äußeren Instrumentenschafts 512 in der Sterileinheit 400. Alternativ zum chirurgischen Instrument 500 lassen sich mit der Sterileinheit 400 auch andere Instrumente, wie eine Schere, Nadelhalter, optische Instrumente, Spüleinheiten, Absaugeinheiten, Instrumente der Hochfrequenzchirurgie und weitere bei Operationen, insbesondere bei laparoskopischen Operationen, eingesetzte Instrumente, koppeln, wobei die zweiten Übertragungsmittel 406 für die Realisierung der entsprechenden Funktionen ausgelegt sind.

Das zweite Übertragungsmittel 406 umfasst gemäß dem Ausführungsbeispiel weiterhin ein elektrisches Übertragungselement mit einem ersten als Schleifring ausgebildeten elektrischen Kontakt 422 und einen zweiten als Schleifring ausgebildeten elektrischen Kontakt 423, die bei einer Kopplung der Sterileinheit 400 mit der Koppeleinheit 100 über die Sterilschleuse 200 mit den elektrischen Kontakten 106, 108 der Koppeleinheit 100 eine elektrische Verbindung zur Übertragung von hochfrequenter elektrischer Energie zur Hochfrequenzchirurgie herstellen. Bei anderen Ausführungsbeispielen können auch keine elektrischen Übertragungsmittel vorgesehen sein.

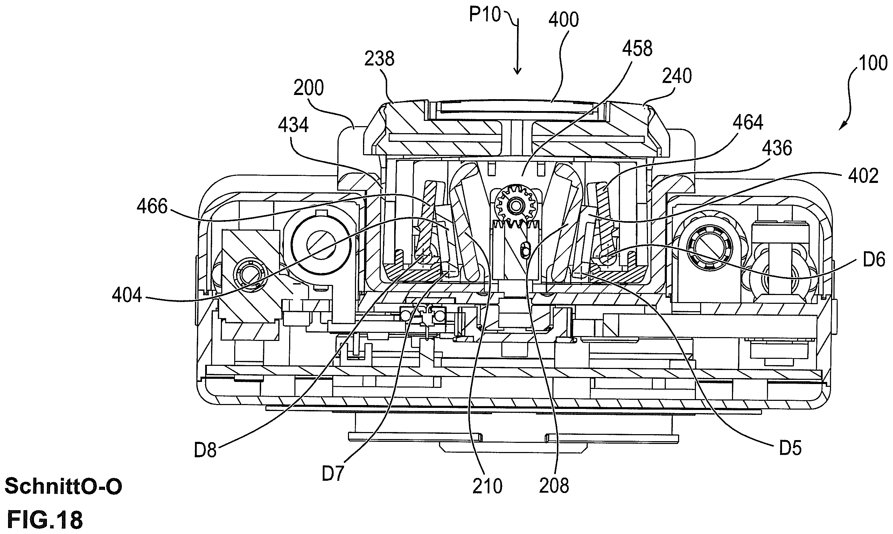

Die Sterileinheit 400 hat zwei vorstehende Nocken 415, 417, die beim Einsetzen der Sterileinheit 400 in die Sterilschleuse 200 die entriegelten Sterilklappen 208, 210 zumindest so weit auseinander drücken, bis die Nocken 415, 417 zwischen den Sterilklappen 208, 210 angeordnet sind. Beim weiteren Einsetzen der Sterileinheit 400 in die Sterilschleuse 200 drücken keilförmige Eingriffselemente 456 bis 462 der Sterileinheit 400 die Sterilklappen 208, 210 weiter auseinander, bis diese in ihrer in Figur 18 gezeigten geöffneten Position angeordnet sind.

Die in den Figuren 11 und 12 nach oben zeigende Bodenplatte 401 der Sterileinheit 400 hat, wie bereits erwähnt, zwei als vorstehende Detektionsstifte ausgebildete Detektionselemente 426, 428. Bei einer Kopplung der Sterileinheit 400 mit der Koppeleinheit 100 mit zwischen der Strileinheit400 und der Koppeleinheit 100 angeordneter Sterilschleuse 200 ragt das Detektionselement 426 durch das erste Detektionsfenster 232 der Sterilschleuse 200 in die Vertiefung des ersten Koppelsensors 118 der Koppeleinheit 100 und das zweite Detektionselement 428 ragt durch das zweite Detektionsfenster 234 in die Vertiefung des zweiten Koppelsensors 120 der Koppeleinheit 100. Bei einer Detektion der Detektionselemente 426, 428 mit Hilfe der Koppelsensoren 118, 120 kann eine korrekte Kopplung der Sterilschleuse 200 mit der Koppeleinheit 100 und der Sterileinheit 400 mit der Sterilschleuse 200 detektiert werden, so dass erst nach einer Detektion der Detektionselemente 426, 428 mit Hilfe der Koppelsensoren 118, 120 ein Antrieb der Übertragungselemente 110 bis 116 von einer Steuereinheit freigegeben wird. Ferner wird die Übertragung von hochfrequenter Energie erst nach der korrekten Detektion der Detektionselemente 426, 428 mit Hilfe der Koppelsensoren 118, 120 über die Übertragungselemente 106, 108 freigegeben.

Die Sterileinheit 400 hat ferner zwei an gegenüberliegenden Seitenwänden 430, 432 angeordnete Rastelemente 434, 436, die mit Hilfe eines aus der Seitenwand 430, 432 vorstehenden Betätigungselements 438, 440 betätigbar sind. Die Rastelemente 434, 436 greifen in die in den Seitenwänden 218, 220 der Sterilschleuse 200 vorgesehenen Rastsicken 250, 252 ein, wenn die Sterileinheit 400 korrekt mit der Sterilschleuse 200 verbunden ist.

Die vordere Stirnwand 442 der Sterileinheit 400 hat zwei Nuten 444, 446, in die die Führungs- und Entriegelungsstege 228, 230 der Sterilschleuse 200 bei einer Verbindung der Sterileinheit 400 mit der Sterilschleuse 200 eingeführt werden und dabei die Sterilklappen 402, 404 entriegeln.

Ferner greift der Führungssteg 254 der Sterilschleuse 200 in die an der hinteren Stirnseite 450 der Sterileinheit 400 vorhandene Führungsnut 452 ein. Am unteren Ende der Führungsnut 452 steht ein Betätigungssteg 454 nach außen aus der Bodenplatte 401 hervor, der beim Einsetzen der Sterileinheit 400 in die Sterilschleuse 200 die Führungsgabel 244 nach unten drückt und dadurch die Verriegelung der Schleusenklappen 208, 210 durch die Führungsgabel 244 löst.

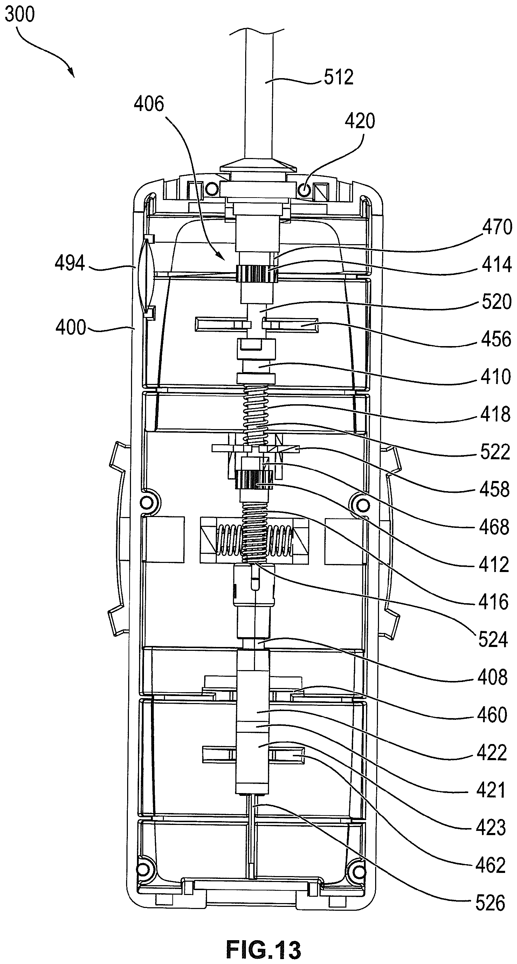

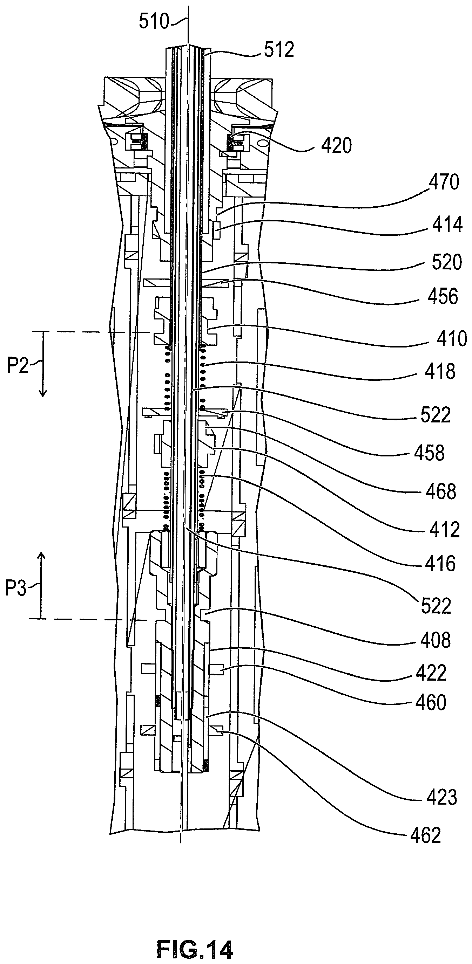

Figur 13 zeigt die Instrumenteneinheit 300 nach den Figuren 11 und 12, wobei die Bodenplatte 401 der Sterileinheit 400 entfernt worden ist. Figur 14 zeigt eine Schnittdarstellung eines Ausschnitts der Instrumenteneinheit 300 nach Figur 13 mit mehreren mithilfe der Antriebselemente 110 bis 116 der Koppeleinheit 100 angetriebenen Elementen 408 bis 414. Das als Zahnrad ausgebildete zweite rotatorische angetriebene Element 414 ist mit dem äußeren Instrumentenschaft 512 und einem zweiten Winkelgeber 470 für die Detektion der Schaftdrehung des äußeren Instrumentenschafts 512 drehfest verbunden. Der zweite Winkelgeber 470 hat an einer Stelle seines Umfangs einen vorstehenden Nocken, der mithilfe eines zweiten Positionssensors 156 der Koppeleinheit 100 detektierbar ist, wenn der Nocken dem zweiten Positionssensor 156 gegenüberliegend angeordnet ist.

Das zweite translatorische angetriebene Element 410 ist mit einem ersten inneren Instrumentenschaft 520 verbunden, sodass bei einer translatorischen Bewegung des zweiten translatorischen angetriebenen Elements 410 der erste innere Instrumentenschaft 520 translatorisch bewegt wird.

Das erste rotatorische angetriebene Element 412 ist drehfest mit einem ersten Winkelgeber 468 und einem zweiten inneren Instrumentenschaft 522 drehfest verbunden. In gleicher Weise wie der zweite Winkelgeber 470 hat der erste Winkelgeber 468 an einer Stelle seines Umfangs einen vorstehenden Nocken, der mithilfe eines ersten Positionssensors 154 der Koppeleinheit 100 detektierbar ist, wenn der Nocken diesem ersten Positionssensor 154 gegenüberliegend angeordnet ist. Der zweite innere Instrumentenschaft 522 dient zur Endeffektordrehung unabhängig von dem Drehwinkel des äußeren Instrumentenschafts 512. Der erste innere Instrumentenschaft 520 dient zur Abwinkelung des Endeffektors 514.

Das erste translatorische angetriebene Element 408 ist mit einem dritten inneren Instrumentenschaft 524 zum Übertragen einer translatorischen Antriebsbewegung verbunden. Der dritte innere Instrumentenschaft 524 dient insbesondere zum Ausführen einer Maulteilbewegung der Greifarme 516, 518 des Endeffektors 514, die insbesondere in Figur 11 dargestellt sind.

Bei einer Initialisierung unmittelbar nach dem Koppeln der Sterileinheit 400 mit der Koppeleinheit 100 erfolgt eine Drehung der rotatorisch angetriebenen Elemente 412, 414 zusammen mit den Winkelgebern 468, 470um jeweils maximal 360°, wodurch die exakte Initialdrehposition des äußeren Instrumentenschafts 512 bzw. des zweiten inneren Instrumentenschafts 522 ermittelt und eingestellt wird, wovon ausgehend die Steuereinheit 38 die exakte Drehposition des jeweiligen Instrumentenschafts 512, 522 ermittelt und mit Hilfe des im jeweiligen Antriebsmotor 144, 146 zugeordneten Tachogenerators kontinuierlich überwacht, sodass die exakte Drehwinkelposition des jeweiligen Instrumentenschafts 512, 522 zu jeder Zeit der Steuereinheit 38 bekannt ist und bei der weiteren Ansteuerung Antriebsmotore 140 bis 146 berücksichtigt werden kann.

Zwischen den als Schleifringen ausgebildeten elektrischen Kontakten 422 und 423 ist eine optische Schnittstelle 421, die auch zur gegenseitigen elektrische Isolierung der elektrischen Kontakte 422, 423 dient. Die elektrischen Kontakte 422, 423 sind mit Kabeln verbunden, die in dem dritten inneren Instrumentenschaft 524 zu den Greifarmen 516, 518 des Endeffektors 514 geführt sind.

Falls erforderlich können durch den dritten inneren Instrumentenschaft 524 weitere Kabel und/oder Lichtleitfasern zum Endeffektor 514 geführt werden.

In der in Figur 14 dargestellten Schnittdarstellung ist die koaxiale Anordnung des äußeren Instrumentenschafts 512 und der inneren Instrumentenschäfte 520 bis 526 gut sichtbar. Die V-förmigen Elemente 456 bis 462 dienen als Lagerungsstege zur Lagerung der inneren Schäfte 520 bis 526, wobei die inneren Schäfte 520 bis 526 direkt oder indirekt drehbar in den Lagerungsstegen 520 bis 526 gelagert sind.

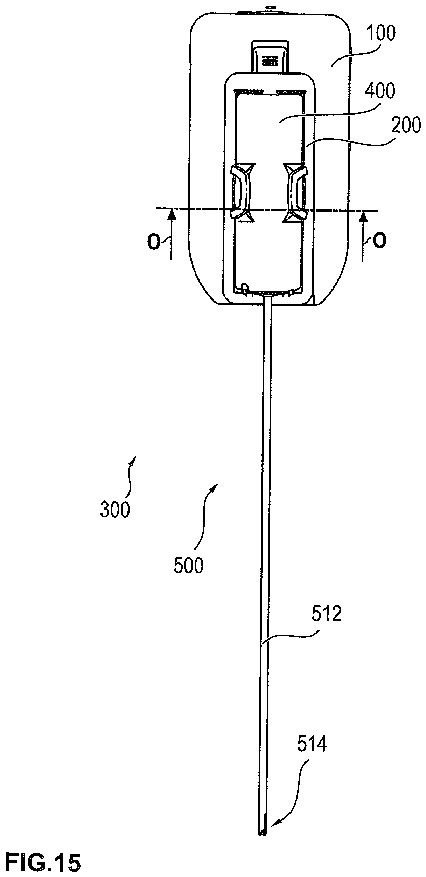

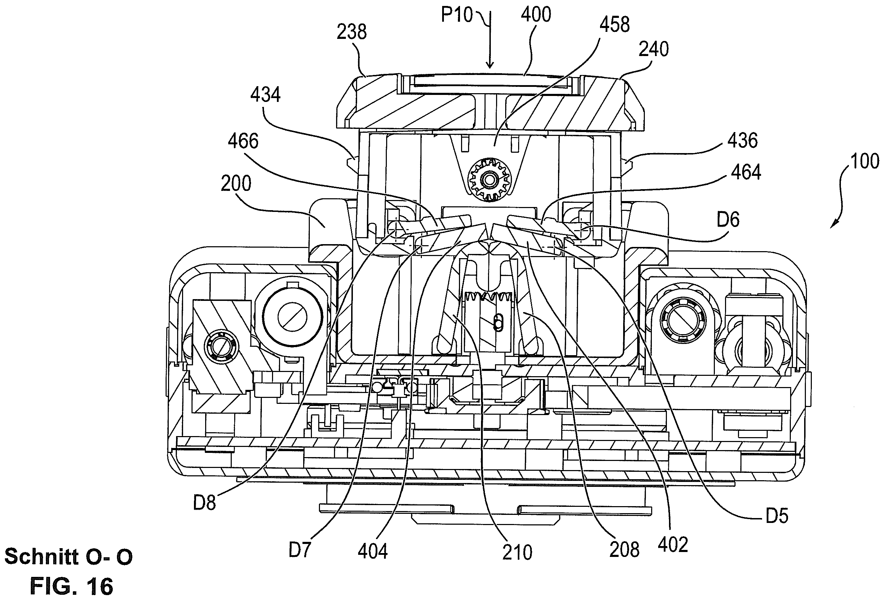

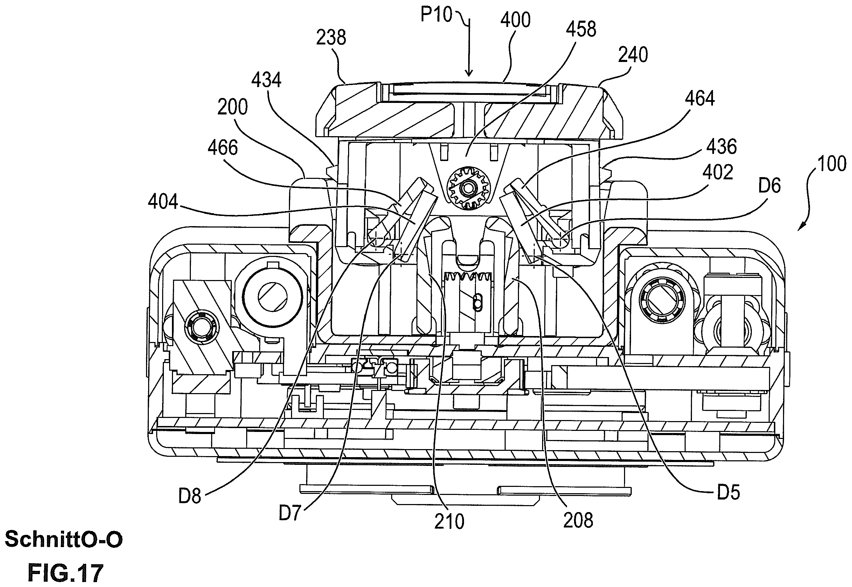

Figur 15 zeigt eine Draufsicht auf eine Anordnung mit der Koppeleinheit 100, der Sterilschleuse 200 und der Instrumenteneinheit 300, welche die sterile Sterileinheit 400 und das sterile chirurgische Instrument 500 umfasst. Figur 16 zeigt eine Schnittdarstellung der Anordnung nach Figur 15 entlang der Schnittlinie O-O in einer ersten Position unmittelbar vor dem Verbinden der Sterileinheit 400 der Instrumenteneinheit 300 mit der bereits mit der Koppeleinheit 100 gekoppelten Sterilschleuse 200. Figur 17 zeigt eine Schnittdarstellung der Anordnung nach Figur 15 entlang der Schnittlinie O-O in einer zweiten Position beim Verbinden der Sterileinheit 400 der Instrumenteneinheit 300 mit der bereits mit der Koppeleinheit 100 gekoppelten Sterilschleuse 200. Figur 18 zeigt eine Schnittdarstellung der Anordnung nach Figur 15 entlang der Schnittlinie O-O in einer dritten Position, in der die Sterileinheit 400 der Instrumenteneinheit 300 mit der mit der Koppeleinheit 100 gekoppelten Sterilschleuse 200 verbunden ist, so dass die ersten Übertragungsmittel 102 der Koppeleinheit 100 zur direkten Koppelung mit den Übertragungselementen der zweiten Übertragungsmittel 406 in Eingriff stehen. Bei der in Figur 16 gezeigten Position sind die Führungsklappen 464, 466 durch das Einführen der Führungs- und Entriegelungsstege 228, 230 in die Nuten 444, 446 bewegt und dadurch bereits entlang ihrer Drehachse D6, D8 in Richtung der Pfeile P4, P5 von ihrer verriegelten Position in ihre entriegelte Position bewegt worden, so dass die Sterilklappen 402, 404 zusammen mit den Führungsklappen 464, 466 durch eine Bewegung der Sterileinheit 400 in Richtung des Pfeils P10 und den dadurch bewirkten Kontakt der Sterilklappen 402, 404 mit den Schleusenklappen 208, 210 aufgedrückt worden sind. Dadurch gelangt die Sterileinheit 400 tiefer in den zur Aufnahme der Sterileinheit 400 vorgesehenen Aufnahmebereich der Sterilschleuse 200, so dass der Betätigungssteg 454 in Eingriff mit der Führungsgabel 244 gelangt und diese entgegen der Federkraft der Führungsgabelfeder verschwenkt. Dadurch stehen die Zinken 246, 248 der Führungsgabel 244 so in Eingriff mit den Führungssicken 240, 242, dass die Schleusenklappen 208, 210 durch die Nocken 415, 417 auseinander gedrückt und dadurch geöffnet werden können. Bei einer weiteren Bewegung der Sterileinheit 400 in Richtung des Pfeils P10 kommen die V-förmigen Eingriffselemente 458 bis 462 in Eingriff mit den Schleusenklappen 208, 210 und drücken diese und die Sterilklappen 402, 404 zusammen mit den Führungsklappen 464, 466 weiter nach außen in ihre in Figur 18 gezeigte voll geöffnete Position. Durch den Kontakt der Schleusenklappen 208, 210 mit den Sterilklappen 402, 404 werden diese zusammen mit den Führungsklappen 464, 466 weiter geöffnet, bis alle Klappen 208, 210, 402, 404, 464, 466 in der in Figur 18 gezeigten geöffneten Position angeordnet sind. Bei einer umgekehrten Bewegung der Sterileinheit 400 beim Entnehmen der Sterileinheit 400 aus der Sterilschleuse 200, d.h. von der in Figur 18 gezeigten Position in die in Figur 16 gezeigte Position entgegen der Richtung des Pfeils P10, erfolgt ein umgekehrter Bewegungsablauf der Steril-, Führungs- und Schleusenklappen, so dass diese Klappen insbesondere durch die Federkraft der Federn geschlossen werden und die Zinken 246, 248 der Führungsgabel 244 wieder in Eingriff mit den Führungssicken 240, 242 gebracht werden und die Schleusenklappe 208, 210 vollständig schließen. Durch den so erzeugten Formschluss der Gabelzinken 246, 248 mit den Führungssicken 240, 244 werden die Schleusenklappen 208, 210 sicher in ihrer geschlossenen Position gehalten, so dass die Schleusenklappen 208, 210 nicht von außen geöffnet werden können. Auch werden die Führungsklappen 464, 466 beim Entfernen der Sterileinheit 400 aus der Sterilschleuse 200 mit Hilfe der Federn vollständig geschlossen und in ihre verriegelte Position bewegt, so dass anschließend auch bei äußerer Krafteinwirkung auf die Sterilklappen 402, 404 diese nicht geöffnet werden können.

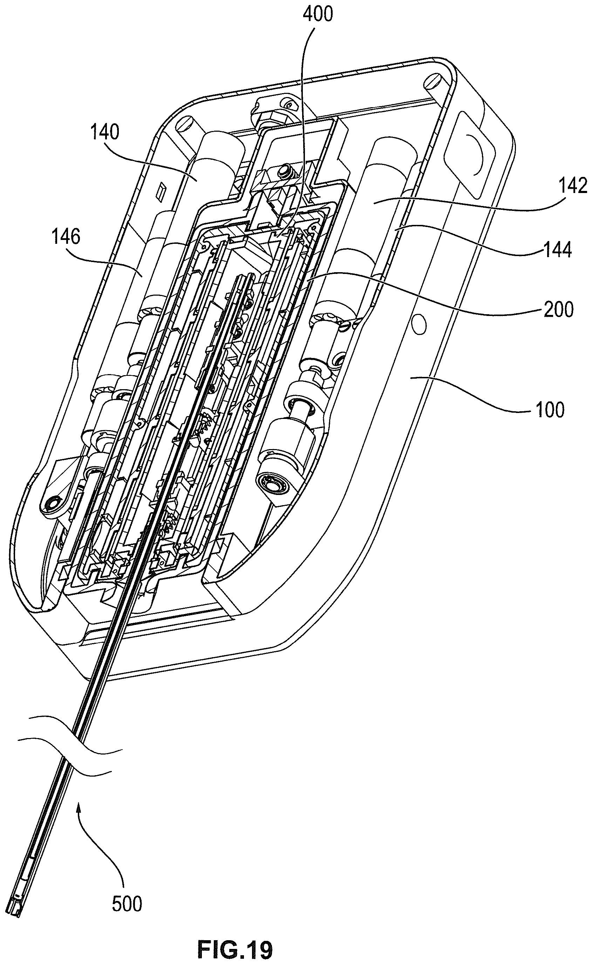

Figur 19 zeigt eine geschnittene Darstellung der Instrumenteneinheit 300 der Sterilschleuse 200 und der Koppeleinheit 100 im verbundenen Zustand. Hierbei sind sowohl von der Koppeleinheit 100 als auch von der Sterileinheit 400 und der Sterilschleuse 200 die oberen Gehäusebereiche abgeschnitten worden, sodass die Anordnungen der Sterilschleuse 200 innerhalb des Aufnahmebereichs der Koppeleinheit 100 und die Anordnung der Sterileinheit 400 innerhalb des Aufnahmebereichs der Sterilschleuse 200 gut sichtbar sind.

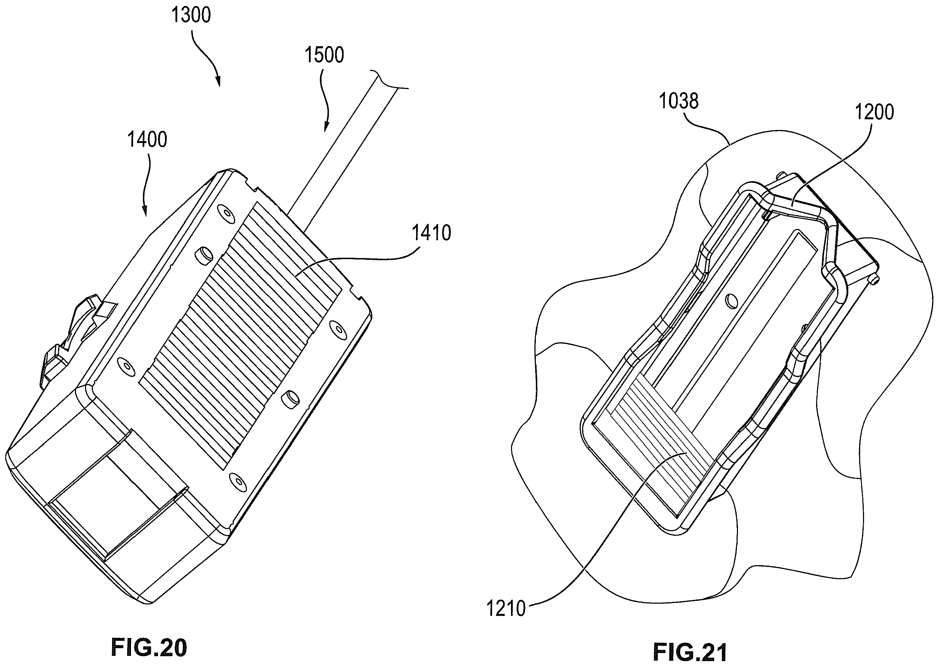

Figur 20 zeigt einen Ausschnitt einer Instrumenteneinheit 1300 mit einer Sterileinheit 1400 und einem chirurgischen Instrument 1500 gemäß einer zweiten Ausführungsform. Im Unterschied zu der Instrumenteneinheit 300 hat die Sterileinheit 1400 der Instrumenteneinheit 1300 keine Sterilklappen sondern eine Jalousie 1410 zum sterilen Abdecken der angetriebenen Elemente. Der weitere Aufbau und die Funktion der Instrumenteneinheit 1300 stimmt mit dem Aufbau und der Funktion der Instrumenteneinheit 300 nach den Figuren 1 bis 19 überein.

Figur 21 zeigt einen Ausschnitt einer sterilen Abdeckung 1038 mit einer Sterilschleuse 1200, die im Unterschied zur Sterilschleuse 200 keine Sterilklappen sondern eine Jalousie 1210 zum sterilen Abschirmen von Antriebselementen der Koppeleinheit 100 hat. Die Jalousie 1410 der Koppeleinheit 1400 als auch die Jalousie 1210 der Sterilschleuse 1200 werden auf geeignete Weise durch einen mechanischen Eingriff beim Verbinden der Sterileinheit 1400 mit der Sterilschleuse 1200 bzw. der Sterileinheit 1400 mit der Sterilschleuse 200 bzw. der Sterileinheit 400 mit der Sterilschleuse 1200 geöffnet. Alternativ können aktive Antriebselemente, wie beispielsweise jeweils ein Elektromotor, zum Öffnen oder Schließen der jeweiligen Jalousie 1410, 1210 vorgesehen sein.

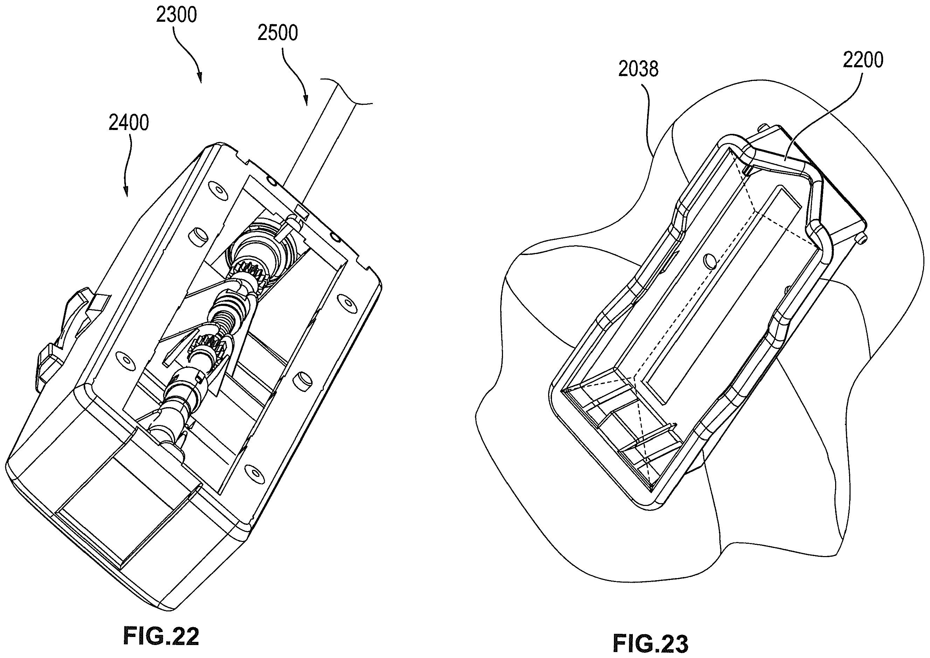

In Figur 22 ist ein Ausschnitt einer Instrumenteneinheit 2300 mit einer Sterileinheit 2400 gemäß einer dritten Ausführungsform gezeigt. Die Sterileinheit 2400 hat keine Elemente zur sterilen Abdeckung der angetriebenen Elemente, sodass diese Instrumenteneinheit 2400 nach dem Trennen von der Sterilschleuse 200, 1200 oder nach dem Trennen von einer weiteren in Figur 23 gezeigten Sterilschleuse 2200 aus dem sterilen Bereich 39 sofort entfernt wird.

Die in Figur 23 gezeigte sterile Abdeckung 2038 umfasst eine Sterilschleuse 2200, die in gleicher Weise wie die Sterileinheit 200 mit der Koppeleinheit 100 verbindbar ist. Im Unterschied zu der Sterilschleuse 200 hat die Sterilschleuse 2200 keine Schleusenklappen sondern umfasst eine mit mithilfe von Punktlinien angedeuteten Sollbruchstellen versehenen Folie, die beim Verbinden einer Sterileinheit 400, 1400, 2400 mit der Sterilschleuse 2200 an den Sollbruchstellen aufgerissen wird, sodass eine direkte Kopplung der Antriebselemente 110 bis 116 der Koppeleinheit 100 mit den angetriebenen Elementen 408 bis 414 einfach möglich ist. Vorzugsweise wird bei einer Verwendung der Sterilschleuse 2200 die Sterileinheit 400, 1400, 2400 während einer Operation nicht getrennt, sondern erst nachdem die Operation beendet worden ist.

### Bezugszeichenliste

- 10: System
- 12: Manipulator
- 14: Stativ
- 16, 16a bis 16d: Manipulatorarm
- 20: Stativkopf
- 22,26: Antriebseinheit
- 24: Stativfuß
- 28, 30: Stativarme
- 32: Operationstischsäule
- 34: Operationstisch
- 36: Steuereinheit
- 37: Ein - und Ausgabeeinheit
- 38: sterile Hülle
- 39: steriler Operationsbereich
- 40a bis 58a, 40b bis 58b, 40c bis 58c, 40d bis 58d: Segmente
- 60: Segment
- 62, 64, 66: Abschnitte der Teleskopanordnung
- 68: Antriebseinheit
- 100: Koppeleinheit
- 102: erstes Übertragungsmittel
- 104: erstes elektrisches Übertragungselement
- 106, 108: elektrischer Kontakt
- 109: optisches Übertragungsmittel
- 110: erstes translatorisches Antriebselement/Linearhubgabel
- 112: zweites translatorisches Antriebselement/Linearhubgabel
- 114: erstes rotatorisches Antriebselement/Antriebsritzel
- 116: zweites rotatorisches Antriebselement/Antriebsritzel
- 118, 120: Koppelsensor
- 121: RFID-Lese_ und Schreibeinheit
- 122, 124: Führungsnut
- 123, 125: vorderes Ende der Führungsnut
- 126: Rastnase
- 128: Rastelement
- 130: Drehachse
- 132: Feder
- 134: Entriegelungstaste
- 136, 138: Öffnung
- 140, 142, 144, 146: Antriebsmotor
- 147: erstes Linearkoppelgetriebe
- 148: zweites Linearkoppelgetriebe
- 150: erste Getriebestufe
- 152: zweite Getriebestufe
- 154, 156: Positionssensor
- 200: Sterilschleuse
- 202: Anschlussrand
- 204, 206: Führungsstift
- 208,210: Schleusenklappe
- 212: Boden
- 214, 216: Öffnung
- 218,220: Seitenwand
- 222: vordere Stirnwand
- 224: hintere Stirnwand
- 226: V-förmige Aussparung
- 228, 230: Führungs- und Entriegelungssteg
- 232,234: Detektionsfenster
- 236 bis 242: Führungssicke
- 244: Führungsgabel
- 246,248: Zinken
- 250, 252: Rastsicke
- 254: Führungssteg
- 266: erster Verbindungsbereich
- 268: zweiter Verbindungsbereich
- 300, 300a bis 300d: Instrumenteneinheit
- 400: Sterileinheit
- 401: Bodenplatte
- 402, 404: Sterilklappe
- 406: zweites Übertragungsmittel
- 408: erstes translatorisches angetriebenes Element
- 410: zweites translatorisches angetriebenes Element
- 412: erstes rotatorisches angetriebenes Element
- 414: zweites rotatorisches angetriebenes Element
- 415, 417: Nocken
- 416, 418: Feder
- 420: Lager des äußeren Instrumentenschafts
- 421: optische Schnittstelle
- 422, 423: elektrischer Kontakt
- 426,428: Detektionselement
- 430, 432: Seitenwand
- 434,436: Rastelement
- 438, 440: Betätigungselement
- 442: vordere Stirnwand
- 444, 446: Nut
- 450: hintere Stirnseite
- 452: Führungsnut
- 454: Betätigungssteg
- 456 bis 462: V-förmiges Eingriffselement
- 464, 466: Führungsklappe
- 468, 470: Winkelgeber
- 494: RFID-Transponder
- 500: Instrument
- 510: Längsachse der Instrumentenschäfte
- 512: äußerer Instrumentenschaft
- 514: Endeffektor
- 516,518: Greifarm
- D4: Kippachse
- D6, D7, D8: Drehachse
- P1, P2, P3: Richtungspfeile
- 520: erster innerer Instrumentenschaft
- 522: zweiter innerer Instrumentenschaft
- 524: dritter innerer Instrumentenschaft
- 1300: Instrumenteneinheit
- 1400: Sterileinheit
- 1500: chirurgisches Instrument
- 1410: Jalousie
- 1038: sterile Abdeckung
- 1200: Sterilschleuse
- 1210: Jalousie
- 2300: Instrumenteneinheit
- 2400: Sterileinheit
- 2500: chirurgisches Instrument
- 2038: sterile Abdeckung
- 2200: Sterilschleuse

## Patentansprüche

1. Vorrichtung zur robotergestützten Chirurgie,
mit mindestens einem nicht sterilen Manipulatorarm (12), der eine Koppeleinheit (100) mit Antriebselementen hat,
mit einer in einem sterilen Bereich angeordneten sterilen Instrumenteneinheit (300), die ein chirurgisches Instrument (500) und eine sterile Sterileinheit (400) zum Koppeln des chirurgischen Instruments (500) mit den Antriebselementen der Koppeleinheit (100) umfasst,
mit einer sterilen Abdeckung (40) zum Abschirmen zumindest eines Teils des Manipulatorarms (12) von dem sterilen Bereich (42), wobei
die Koppeleinheit (100) ein translatorisches Antriebselement (110, 112) zum Erzeugen einer translatorischen Antriebsbewegung und ein rotatorisches Antriebselement (114, 116) zum Erzeugen einer rotatorischen Antriebsbewegung umfasst,
die Sterileinheit (400) ein mit dem translatorischen Antriebselement (110, 112) koppelbares translatorisches angetriebenes Element (408, 410) und ein mit dem rotatorischen Antriebselement (114, 116) koppelbares rotatorisches angetriebenes Element (412, 414) hat, **dadurch gekennzeichnet,**
**dass** die sterile Abdeckung (40) eine Sterilschleuse (200) umfasst, die mit der Koppeleinheit (100) und mit der Sterileinheit (400) verbindbar ist,
**dass** die mit der Koppeleinheit (100) verbundene Sterilschleuse (200) das translatorische Antriebselement (110, 112) und das rotatorische Antriebselement (114, 116) steril abschirmt, bevor die Sterileinheit (400) mit der Sterilschleuse (200) verbunden ist und nachdem die Sterileinheit (400) von der Sterilschleuse (200) getrennt worden ist.

2. Vorrichtung nach Anspruch 1, **dadurch gekennzeichnet, dass** die mit der Koppeleinheit (100) und mit der Sterileinheit (400) verbundene Sterilschleuse (200) einen Zugang zu den Antriebselementen (110 bis 116)derart freigibt, dass das translatorische Antriebselement (110, 112) mit den translatorischen angetriebenen Element (408, 410) und das rotatorische Antriebselement (114, 116) mit dem rotatorischen angetriebenen Element (412, 414) koppelbar ist.

3. Vorrichtung nach einem der vorhergehenden Ansprüche, **dadurch gekennzeichnet, dass** die Sterileinheit (400) eine sterile Abdeckung (402,404) umfasst, die die angetriebenen Elemente (408 bis 414) steril abschirmt, bevor die Sterileinheit (400) mit der Sterilschleuse (200) verbunden ist und nachdem die Sterileinheit (400) von der Sterilschleuse (200) getrennt worden ist, und dass die Sterileinheit (400) den Zugang zu den angetriebenen Elementen (408 bis 414) freigibt, wenn die Sterilschleuse (200) mit der Koppeleinheit (100) und mit der Sterileinheit (400) verbunden ist.

4. Vorrichtung nach einem der vorgehrgehenden Ansprüche, **dadurch gekennzeichnet, dass** die Sterilschleuse (200) eine sterile Abdeckung (208, 210) umfasst, die die Antriebselemente (110 bis 116) der Koppeleinheit (100) steril abschirmt, bevor die Sterileinheit (400) mit der Sterilschleuse (200) verbunden ist und nachdem die Sterileinheit (400) von der Sterilschleuse (200) getrennt worden ist, und dass die Sterilschleuse (200) den Zugang zu den Antriebselementen (110 bis 116) freigibt, wenn die Sterilschleuse (200) mit der Koppeleinheit (100) und mit der Sterileinheit (400) verbunden ist.

5. Vorrichtung nach Anspruch 3 oder 4, **dadurch gekennzeichnet, dass** die sterile Abdeckung eine Jalousie (1410), ein Rollo, eine mit einer Öffnung versehenen drehbare Scheibe und/oder eine Klappe (208, 210, 402, 404) umfasst.

6. Vorrichtung nach einem der vorhergehenden Ansprüche, **dadurch gekennzeichnet, dass** das translatorische Antriebselement (110) der Koppeleinheit (100) ein erstes translatorisches Antriebselement (110) und das rotatorische Antriebselement (114) der Koppeleinheit (100) ein erstes rotatorisches Antriebselement (114) ist,
dass das translatorische angetriebene Element (408) der Sterileinheit (400) ein erstes translatorisches angetriebenes Element (408) und dass das rotatorische angetriebene Element (412) ein erstes rotatorisches angetriebenes Element (412) ist,
dass die Koppeleinheit (100) ein zweites translatorisches Antriebselement (112) zum Erzeugen einer translatorischen Antriebsbewegung und ein zweites rotatorisches Antriebselement (116) zum Erzeugen einer rotatorischen Antriebsbewegung umfasst,
dass die Sterileinheit (400) ein mit dem zweiten translatorischen Antriebselement (112) koppelbares zweites translatorisches angetriebenes Element (410) und ein mit dem zweiten rotatorischen Antriebselement (116) koppelbares zweites rotatorisches angetriebenes Element (414) hat,
dass die mit der Koppeleinheit (100) verbundene Sterilschleuse (200) das erste und das zweite translatorische Antriebselement (110, 112) und das erste und das zweite rotatorische Antriebselement (114, 116) steril abschirmt, bevor die Sterileinheit (400) mit der Sterilschleuse (200) verbunden ist und nachdem die Sterileinheit (400) von der Sterilschleuse (200) getrennt worden ist.

7. Vorrichtung nach einem der vorhergehenden Ansprüche, **dadurch gekennzeichnet, dass** das erste und/oder zweite translatorische angetriebene Element (408, 410) mit Hilfe der Rückstellkraft eines elastisch verformbaren Elements (416, 418) in eine Ausgangsposition bewegbar ist, und dass diese Rückstellkraft das jeweilige translatorische angetriebene Element (408, 410) nach dem Trennen der Sterileinheit (400) von der Sterilschleuse (200) in seine Ausgangsposition bewegt.

8. Vorrichtung nach einem der vorhergehenden Ansprüche, **dadurch gekennzeichnet, dass** die Koppeleinheit (100) mindestens einen Positionssensor (154, 156) zum Detektieren mindestens einer Drehwinkelposition des ersten und/oder zweiten rotatorisch angetriebenen Elements (412, 414) hat.

9. Vorrichtung nach einem der vorhergehenden Ansprüche, **dadurch gekennzeichnet, dass** die Koppeleinheit für jedes Antriebselement (110 bis 116) eine separate Antriebseinheit (140 bis 146) aufweist.

10. Vorrichtung nach einem der vorhergehenden Ansprüche, **dadurch gekennzeichnet, dass** die Koppeleinheit (100) das translatorische Antriebselement (110, 112) in eine Ausgansposition bewegt, nachdem die Sterileinheit (400) von der Sterilschleuse (200) getrennt worden ist, wobei das in seiner Ausgangsposition angeordnete translatorische Antriebselement (110, 112) mit dem in seiner Ausgangsposition angeordneten translatorischen angetriebenen Element (408, 410) beim Verbinden der Sterileinheit (400) mit der mit der Koppeleinheit (100) verbundenen Sterilschleuse (200) automatisch in direkten Eingriff gebracht wird.

11. Vorrichtung nach einem der vorhergehenden Ansprüche, **dadurch gekennzeichnet, dass** die angetriebenen Elemente (408 bis 414) der Sterileinheit (400) entlang der Längsachse (510) des chirurgischen Instruments (500) nacheinander angeordnet sind,
wobei die angetriebenen Elemente (408 bis 414) von dem proximalen Ende des chirurgischen Instruments (500) in der Reihenfolge zweites rotatorisches angetriebenes Element (414), zweites translatorisches angetriebenes Element (410), erstes rotatorisches angetriebenes Element (412), erstes translatorisches angetriebenes Element (408) und nachfolgend optional elektrische Kontakte (422, 424) und/oder eine optische Schnittstelle (472) angeordnet sind, wobei die Koppeleinheit (100) komplementäre Antriebselemente (108 bis 116), elektrische Kontakte (106, 108) und/oder eine optische Schnittstelle (109) umfasst.

12. Vorrichtung nach einem der vorhergehenden Ansprüche, **dadurch gekennzeichnet, dass** das zweite rotatorische angetriebene Element (414) drehfest mit dem äußeren Instrumentenschaft (512) zu dessen Drehung verbunden ist,
dass das zweite translatorische angetriebene Element (410) mit einem in dem äußeren Instrumentenschaft (512) in Richtung der Längsachse (510) des äußeren Instrumentenschafts (512) längs verschiebbar angeordneten ersten inneren Instrumentenschaft (520) verbunden ist, wobei bei einer Bewegung des ersten inneren Instrumentenschafts (520) ein Endeffektor (514) des Chirurgischen Instruments (500) um eine Kippachse (D4) schwenkbar ist,
dass das erste rotatorische angetriebene Element (412) drehfest mit einem in dem ersten inneren Instrumentenschaft (520) angeordneten zweiten inneren Instrumentenschaft (522) verbunden ist, wobei bei Drehung des zweiten inneren Instrumentenschafts (522) eine Rotation des Endeffektors (514) unabhängig von dem äußeren Instrumentenschaft (512) erfolgt,
dass das erste translatorische angetriebene Element (408) mit einem relativ zum zweiten inneren Instrumentenschaft (522) in diesem längs verschiebbar angeordneten dritten inneren Instrumentenschaft (524) zum Betätigen von relativ zueinander bewegbaren Armen (516, 518) des Endeffektors (514) verbunden ist.

13. Vorrichtung nach Anspruch 12, **dadurch gekennzeichnet, dass** die Arme (516, 518) des Endeffektors (514) Schneidarme, Greifarme und/oder Nadelhalterarme sind.

14. Vorrichtung nach Anspruch 12 oder 13, **dadurch gekennzeichnet, dass** die Arme (516, 518) des Endeffektors (514) elektrisch isoliert zueinander angeordnet sind,
dass die Arme (516, 518) mit jeweils einem Kabel elektrisch leitend verbunden sind, die durch den dritten inneren Instrumentenschaft (524) geführt sind.

15. Anordnung zur robotergestützten Chirurgie, insbesondere zu einer telerobotergestützten Prozedur innerhalb eines sterilen Bereichs (39),
mit mindestens einer Vorrichtung nach einem der vorhergehenden Ansprüche 1 bis 14,
mit mindestens einer Anzeigeeinheit, die mindestens ein Bild des Operationsgebiets in Echtzeit ausgibt,
mit mindestens einer Eingabeeinrichtung (37) zur Eingabe mindestens eines Eingabekommandos,
mit einer Steuereinheit (9), die den Manipulatorarm (16) und die über die Sterilschleuse (200) mit der Koppeleinheit (100) des Manipulatorarms (16) verbundene Sterileinheit (400) abhängig von dem Eingabekommando mit Hilfe mindestens einer Antriebseinheit positioniert.

## Claims

1. A device for robot-assisted surgery, comprising
at least one non-sterile manipulator arm (12) having a coupling unit (100) with drive elements,
a sterile instrument unit (300) arranged in a sterile area and comprising a surgical instrument (500) and a sterile sterile unit (400) for coupling the surgical instrument (500) to the drive elements of the coupling unit (100),
a sterile cover (40) for shielding at least a part of the manipulator arm (12) from the sterile area (42), wherein
the coupling unit (100) comprises a translatory drive element (110, 112) for generating a translatory drive movement and a rotatory drive element (114, 116) for generating a rotatory drive movement,
the sterile unit (400) has a translationally driven element (408, 410) couplable to the translatory drive element (110, 112) and a rotationally driven element (412, 414) couplable to the rotatory drive element (114, 116), **characterized in**
**that** the sterile cover (40) comprises a sterile lock (200) which is connectable to the coupling unit (100) and to the sterile unit (400), that the sterile lock (200) connected to the coupling unit (100) shields the translatory drive element (110, 112) and the rotatory drive element (114, 116) in a sterile manner before the sterile unit (400) is connected to the sterile lock (200) and after the sterile unit (400) has been separated from the sterile lock (200).

2. The device according to claim 1, **characterized in that** the sterile lock (200) connected to the coupling unit (100) and to the sterile unit (400) provides access to the drive elements (110 to 116) such that the translatory drive element (110, 112) is couplable to the translationally driven element (408, 410) and the rotatory drive element (114, 116) is couplable to the rotationally driven element (412, 414).

3. The device according to one of the preceding claims, **characterized in that** the sterile unit (400) comprises a sterile cover (402, 404) which shields the driven elements (408 to 414) in a sterile manner before the sterile unit (400) is connected to the sterile lock (200) and after the sterile unit (400) has been separated from the sterile lock (200) and that the sterile unit (400) provides access to the driven elements (408 to 414) when the sterile lock (200) is connected to the coupling unit (100) and to the sterile unit (400).

4. The device according to one of the preceding claims, **characterized in that** the sterile lock (200) comprises a sterile cover (208, 210) which shields the drive elements (110 to 116) of the coupling unit (100) in a sterile manner before the sterile unit (400) is connected to the sterile lock (200) and after the sterile unit (400) has been separated from the sterile lock (200), and that the sterile lock (200) provides access to the drive elements (110 to 116) when the sterile lock (200) is connected to the coupling unit (100) and to the sterile unit (400).

5. The device according to claim 3 or 4, **characterized in that** the sterile cover comprises a jalousie (1410), a roller blind, a rotatable disk provided with an opening and/or a flap (208, 210, 402, 404).

6. The device according to one of the preceding claims, **characterized in that** the translatory drive element (110) of the coupling unit (100) is a first translatory drive element (110) and the rotatory drive element (114) of the coupling unit (100) is a first rotatory drive element (114),
that the translationally driven element (408) of the sterile unit (400) is a first translationally driven element (408) and that the rotationally driven element (412) is a first rotationally driven element (412),
that the coupling unit (100) comprises a second translatory drive element (112) for generating a translatory drive movement and a second rotatory drive element (116) for generating a rotatory drive movement,
that the sterile unit (400) has a second translationally driven element (410) couplable to the second translatory drive element (112) and a second rotationally driven element (414) couplable to the second rotatory drive element (116),
that the sterile lock (200) connected to the coupling unit (100) shields the first and the second translatory drive element (110, 112) and the first and second rotatory drive element (114, 116) in a sterile manner before the sterile unit (400) is connected to the sterile lock (200) and after the sterile unit (400) has been separated from the sterile lock (200).

7. The device according to one of the preceding claims, **characterized in that** the first and/or second translationally driven element (408, 410) is movable into an initial position by means of the restoring force of an elastically deformable element (416, 418), and that this restoring force moves the respective translationally driven element (408, 410) into its initial position after separating the sterile unit (400) from the sterile lock (200).

8. The device according to one of the preceding claims, **characterized in that** the coupling unit (100) has at least one position sensor (154, 156) for detecting at least one rotary angle position of the first and/or second rotationally driven element (412, 414).

9. The device according to one of the preceding claims, **characterized in that** the coupling unit has a separate drive unit (140 to 146) for each drive element (110 to 116).

10. The device according to one of the preceding claims, **characterized in that** the coupling unit (100) moves the translatory drive element (110, 112) into an initial position after the sterile unit (400) has been separated from the sterile lock (200), wherein the translatory drive element (110, 112) arranged in its initial position is automatically brought into direct engagement with the translationally driven element (408, 410) arranged in its initial position when connecting the sterile unit (400) to the sterile lock (200) that is connected to the coupling unit (100).

11. The device according to one of the preceding claims, **characterized in that** the driven elements (408 to 414) of the sterile unit (400) are arranged successively along the longitudinal axis (510) of the surgical instrument (500),
wherein the driven elements (408 to 414) are arranged, starting from the proximal end of the surgical instrument (500), in the order second rotationally driven element (414), second translationally driven element (410), first rotationally driven element (412), first translationally driven element (408) and thereafter optionally electrical contacts (422, 424) and/or an optical interface (472), wherein the coupling unit (100) comprises complementary drive elements (108 to 116), electrical contacts (106, 108) and/or an optical interface (109).

12. The device according to one of the preceding claims, **characterized in that** the second rotationally driven element (414) is connected in a rotationally fixed manner to the outer instrument shaft (512) for rotation thereof,
that the second translationally driven element (410) is connected to a first inner instrument shaft (520) arranged so as to be movable lengthwise in the outer instrument shaft (512) in the direction of the longitudinal axis (510) of the outer instrument shaft (512), wherein, when the first inner instrument shaft (520) is moved, an end effector (514) of the surgical instrument (500) is pivotable about a tilt axis (D4), that the first rotationally driven element (412) is connected in a rotationally fixed manner to a second inner instrument shaft (522) arranged in the first inner instrument shaft (520), wherein, when the second inner instrument shaft (522) is rotated, a rotation of the end effector (514) is caused independent of the outer instrument shaft (512),
that the first translationally driven element (408) is connected to a third inner instrument shaft (524) arranged so as to be movable lengthwise relative to the second inner instrument shaft (522) in the second inner instrument shaft for actuating arms (516, 518) of the end effector (514) which are movable relative to each other.

13. The device according to claim 12, **characterized in that** the arms (516, 518) of the end effector (514) are cutting arms, gripping arms, and/or needle holder arms.

14. The device according to claim 12 or 13, **characterized in that** the arms (516, 518) of the end effector (514) are arranged electrically insulated to each other,
that the arms (516, 518) are each connected in an electrically conductive manner to one cable, which cables are passed through the third inner instrument shaft (524).

15. An arrangement for robot-assisted surgery, in particular for a telerobot-assisted procedure within a sterile area (39), comprising
at least one device according to one of the preceding claims 1 to 14,
at least one display unit which outputs at least one image of the operating area in real time,
at least one input device (37) for the input of at least one input command,
a control unit (9) which positions the manipulator arm (16) and the sterile unit (400) connected via the sterile lock (200) to the coupling unit (100) of the manipulator arm (16) dependent on the input command by means of at least one drive unit.

## Revendications

1. Dispositif de chirurgie robotisé,
comprenant au moins un bras manipulateur (12) non stérile, qui présente une unité d'accouplement (100) pourvue d'éléments d'entraînement,
un ensemble d'instruments stériles (300) agencé dans une zone stérile et qui comporte un instrument chirurgical (500) et une unité stérile (400) permettant l'accouplement de l'instrument chirurgical (500) aux éléments d'entraînement de l'unité d'accouplement (100),
un capot stérile (40) permettant de protéger au moins une partie du bras manipulateur (12) de la zone stérile (42),
l'unité d'accouplement (100) comprenant un élément d'entraînement en translation (110, 112) destiné à produire un mouvement d'entraînement en translation et un élément d'entraînement en rotation (114, 116) destiné à produire un mouvement d'entraînement en rotation,
l'unité stérile (400) présentant un élément entraîné en translation (408, 410) pouvant être accouplé à l'élément d'entraînement en translation (110, 112) et un élément entraîné en rotation (412, 414) pouvant être accouplé à l'élément d'entraînement en rotation (114, 116), **caractérisé en ce que**
le capot stérile (40) comprend un sas stérile (200) qui peut être relié à l'unité d'accouplement (100) et à l'unité stérile (400),
**en ce que** le sas stérile (200) relié à l'unité d'accouplement (100) protège de manière stérile l'élément d'entraînement en translation (110, 112) et l'élément d'entraînement en rotation (114, 116), avant que l'unité stérile (400) ne soit reliée au sas stérile (200) et une fois l'unité stérile (400) séparée du sas stérile (200).

2. Dispositif selon la revendication 1, **caractérisé en ce que** le sas stérile (200) relié à l'unité d'accouplement (100) et à l'unité stérile (400) libère un accès aux éléments d'entraînement (110 à 116), de telle sorte que l'élément d'entraînement en translation (110, 112) peut être accouplé à l'élément entraîné en translation (408, 410) et l'élément d'entraînement en rotation (114, 116) peut être accouplé à l'élément entraîné en rotation (412, 414).

3. Dispositif selon l'une quelconque des revendications précédentes, **caractérisé en ce que** l'unité stérile (400) comprend un capot stérile (402, 404), qui protège de manière stérile les éléments entraînés (408 à 414), avant que l'unité stérile (400) ne soit reliée au sas stérile (200) et une fois l'unité stérile (400) séparée du sas stérile (200), et **en ce que** l'unité stérile (400) libère l'accès aux éléments entraînés (408 à 414), lorsque le sas stérile (200) est relié à l'unité d'accouplement (100) et à l'unité stérile (400).

4. Dispositif selon l'une quelconque des revendications précédentes, **caractérisé en ce que** le sas stérile (200) comprend un capot stérile (208, 210), qui protège de manière stérile les éléments d'entraînement (110 à 116) de l'unité d'accouplement (100), avant que l'unité stérile (400) n'ait été reliée au sas stérile (200) et une fois le sas stérile (200) séparé du sas stérile (200), et **en ce que** le sas stérile (200) libère l'accès aux éléments d'entraînement (110 à 116) lorsque le sas stérile (200) est relié à l'unité d'accouplement (100) et à l'unité stérile (400).

5. Dispositif selon la revendication 3 ou 4, **caractérisé en ce que** le capot stérile englobe une jalousie (1410), un store, un disque rotatif pourvu d'une ouverture et/ou un clapet (208, 210, 402, 404).

6. Dispositif selon l'une quelconque des revendications précédentes, **caractérisé en ce que** l'élément d'entraînement en translation (110) de l'unité d'accouplement (100) est un premier élément d'entraînement en translation (110) et l'élément d'entraînement en rotation (114) de l'unité d'accouplement (100) est un premier élément d'entraînement en rotation (114),
**en ce que** l'élément entraîné en translation (408) de l'unité stérile (400) est un premier élément entraîné en translation (408) et **en ce que** l'élément entraîné en rotation (412) est un premier élément entraîné en rotation (412),
**en ce que** l'unité d'accouplement (100) comprend un deuxième élément d'entraînement en translation (112) destiné à produire un mouvement d'entraînement en translation et un deuxième élément d'entraînement en rotation (116) destiné à produire un mouvement d'entraînement en rotation,
**en ce que** l'unité stérile (400) présente un deuxième élément entraîné en translation (410) pouvant être accouplé au deuxième élément d'entraînement en translation (112) et un deuxième élément entraîné en rotation (414) pouvant être accouplé au deuxième élément d'entraînement en rotation (116),
**en ce que** le sas stérile (200) relié à l'unité d'accouplement (100) protège de manière stérile le premier et le deuxième élément d'entraînement en translation (110, 112) et le premier et le deuxième élément d'entraînement en rotation (114, 116), avant que l'unité stérile (400) n'ait été reliée au sas stérile (200) et une fois l'unité stérile (400) séparée du sas stérile (200).

7. Dispositif selon l'une quelconque des revendications précédentes, **caractérisé en ce que** le premier et/ou le deuxième élément entraîné en translation (408, 410) peuvent être déplacés dans une position de départ à l'aide de la force de rappel d'un élément déformable élastiquement (416, 418), et **en ce que** cette force de rappel déplace l'élément entraîné en translation (408, 410) respectif dans sa position de départ une fois l'unité stérile (400) séparée du sas stérile (200).

8. Dispositif selon l'une quelconque des revendications précédentes, **caractérisé en ce que** l'unité d'accouplement (100) présente au moins un capteur de position (154, 156) destiné à détecter au moins une position d'angle de rotation du premier et/ou du deuxième élément entraîné en rotation (412, 414).

9. Dispositif selon l'une quelconque des revendications précédentes, **caractérisé en ce que** l'unité d'accouplement comprend pour chaque élément d'entraînement (110 à 116) une unité d'entraînement (140 à 146) séparée.

10. Dispositif selon l'une quelconque des revendications précédentes, **caractérisé en ce que** l'unité d'accouplement (100) déplace l'élément d'entraînement en translation (110, 112) dans une position de départ, une fois l'unité stérile (400) séparée du sas stérile (200), l'élément d'entraînement en translation (110, 112) agencé dans sa position de départ étant amené automatiquement en contact direct avec l'élément entraîné en translation (408, 410) agencé dans sa position de départ lors de la liaison de l'unité stérile (400) au sas stérile (200) relié à l'unité d'accouplement (100).

11. Dispositif selon l'une quelconque des revendications précédentes, **caractérisé en ce que** les éléments entraînés (408 à 414) de l'unité stérile (400) sont disposés les uns derrière les autres le long de l'axe longitudinal (510) de l'instrument chirurgical (500),
les éléments entraînés (408 à 414) étant agencés de l'extrémité proximale de l'instrument chirurgical (500) dans cet ordre, deuxième élément entraîné en rotation (414), deuxième élément entraîné en translation (410), premier élément entraîné en rotation (412), premier élément entraîné en translation (408) puis éventuellement des contacts électriques (422, 424) et/ou une interface optique (472), l'unité d'accouplement (100) comprenant des éléments d'entraînement (108 à 116) complémentaires, des contacts électriques (106, 108) et/ou une interface optique (109).

12. Dispositif selon l'une quelconque des revendications précédentes, **caractérisé en ce que** le deuxième élément entraîné en rotation (414) est relié de manière solidaire en rotation à la tige d'instrument extérieure (512) pour faire tourner celle-ci,
**en ce que** le deuxième élément entraîné en translation (410) est relié à une première tige d'instrument intérieure (520) agencée de manière à pouvoir se déplacer longitudinalement dans la tige d'instrument extérieure (512) en direction de l'axe longitudinal (510) de la tige d'instrument extérieure (512), un effecteur terminal (514) de l'instrument chirurgical (500) pouvant pivoter autour d'un axe de basculement (D4) lors d'un déplacement de la première tige d'instrument intérieure (520),
**en ce que** le premier élément entraîné en rotation (412) est relié de manière solidaire en rotation à une deuxième tige d'instrument intérieure (522) agencée dans la première tige d'instrument intérieure (520), une rotation de l'effecteur terminal (514) étant réalisée indépendamment de la tige d'instrument extérieure (512) lors de la rotation de la deuxième tige d'instrument intérieure (522),
**en ce que** le premier élément entraîné en translation (408) est relié à une troisième tige d'instrument intérieure (524) agencée de manière à pouvoir se déplacer longitudinalement par rapport à la deuxième tige d'instrument intérieure (522) dans celle-ci, afin d'actionner les bras (516, 518) mobiles l'un par rapport à l'autre de l'effecteur terminal (514).

13. Dispositif selon la revendication 12, **caractérisé en ce que** les bras (516, 518) de l'effecteur terminal (514) sont des bras de coupe, des bras de préhension et/ou des bras porte-aiguille.

14. Dispositif selon la revendication 12 ou 13, **caractérisé en ce que** les bras (516, 518) de l'effecteur terminal (514) sont agencés en étant isolés électriquement les uns des autres,
**en ce que** les bras (516, 518) sont reliés de manière électro-conductrice à un câble respectif, qui sont guidés à travers la troisième tige d'instrument intérieure (524).

15. Ensemble de chirurgie robotisé, permettant en particulier une procédure robotisée à distance à l'intérieur d'une zone stérile (39),
comprenant au moins un dispositif selon l'une quelconque des revendications 1 à 14,
au moins une unité d'affichage, qui délivre au moins une image du domaine d'opération en temps réel,
au moins un système d'entrée (37) destiné à entrer au moins une commande d'entrée,
une unité de commande (9), qui positionne le bras manipulateur (16) et l'unité stérile (400) reliée à l'unité d'accouplement (100) du bras manipulateur (16) par l'intermédiaire du sas stérile (200) en fonction de la commande d'entrée à l'aide d'au moins une unité d'entraînement.
